# EUROPEAN PATENT APPLICATION

(11) **EP 4 068 306 A1**
(43) Date of publication of application: **05.10.2022**
(21) Application number: 20891831.8
(22) Date of filing: 27.11.2020
(51) Int. Cl.: G16H 50/80, G16H 50/50, G16H 70/60, G16H 80/00

(54) **METHOD AND DEVICE FOR CALCULATING PROBABILITY OF BEING INFECTED WITH OR HAVING DISEASE, AND METHOD AND DEVICE FOR OUTPUTTING SUBJECT TO BE TESTED FOR DISEASE**

(30) Priority: 29.11.2019 KR 20190157620; 18.12.2019 KR 20190169920
(71) Applicant: Industry-Academic Cooperation Foundation, Yonsei University, Seoul 03722 (KR)
(72) Inventor: KIM, Sung Won, Yongin-si Gyeonggi-do 16875 (KR); PARK, Jin Young, Seoul 08011 (KR); KIM, Soo Jeong, Seoul 06004 (KR); CHOI, Dong Hoon, Seoul 04385 (KR); SON, Nak Hoon, Seongnam-si Gyeonggi-do 13646 (KR); PARK, Yoon Soo, Goyang-si Gyeonggi-do 10416 (KR)
(74) Representative: Fabry, Bernd
(86) International application number: PCT/KR2020/017157
(87) International publication number: WO 2021/107709

(57) **Abstract**

Disclosed are a method and device for calculating the probability of being infected with or having a disease and a method and device for outputting a subject to be tested for a disease. The method for calculating the probability of being infected with a disease according to one embodiment of the present invention, is a method carried out on a computing device provided with one or more processors, and memory storing one or more programs executed by the one or more processors, the method comprising the steps of: receiving location information of each of a plurality of persons; and calculating the probability of being infected with a disease for each of the plurality of persons, on the basis of infected person information including identification information thereof and disease information thereof, and location information of each of the plurality of persons.

## Description

### [Technical Field]

Disclosed exemplary embodiments relate to a method and device for calculating the probability of being infected with or having a disease and a method and device for outputting a subject to be tested for a disease. More particularly, the disclosed exemplary embodiments relate to a method for calculating a probability of being infected with a disease to calculate a probability of being infected with a disease of each of a plurality of persons, a method and device for outputting a subject to be tested for a disease to determine a subject to be tested for a disease.

### [Background Art]

The MERS (MERS-CoV, Middle East Respiratory Syndrome, 2012) outbreak in the summer of 2015 was an event confirmed once again that the risk of a new infectious disease brought social and economic shocks and chaos equivalent to the national disaster situation such as the collapse of Seongsu Bridge in 1994, the collapse of Sampoong Department Store in 1995, and the sinking of MV Sewol in 2014. From the first confirmed case of the MERS infection in Korea on May 20, 2015 to the declaration of the end of the MERS in July 28, a total of 36 deaths and 186 confirmed cases occurred and about 16000 people were quarantined. In the midst of such nationwide chaos, the estimated economic loss due to the shrinking of social and economic activity also reached 10 trillion won, and the entire nation suffered from socio-psychological and economic aftereffects.

The MERS virus has a higher fatality rate than the influenza virus, but the human-to-human infectivity is low. It is understood that the main causes of the infection route of the MERS virus confirmed so far are droplet infection and direct or indirect physical contact with the infected patients. Also, the possibility of the infection through airborne transmission cannot be excluded. Further, it was understood that the majority of the MERS patients were infected through visits to the hospital facilities or direct or indirect contact with the confirmed patients.

Generally, such infectious diseases may be spread by people who move. Accordingly, in order to prevent the infectious diseases and prevent the spread of the infectious diseases, it is necessary to understand the spread state of the infectious diseases and analyze the spread prediction.

The background of the present disclosure is described for easier understanding of the present invention. It should not be understood to admit the matters described in the background of the present invention as a prior art.

### [Disclosure]

### [Technical Problem]

An object of the disclosed exemplary embodiments is to provide a method and device for calculating a probability of being infected with or having a disease that can contribute to preventing the infectious disease and preventing the spread of the infectious disease as described above. Such a method and device may calculate a probability of being infected with or having a disease of each of the plurality of persons centered on the infected person.

An object of the disclosed exemplary embodiments is to provide a method and device for outputting a subject to be tested for a disease that determine a subject to be tested for a disease, and output information related to the subject to be tested for a disease. Such a method and device may contribute to preventing the infectious disease and preventing the spread of the infectious disease as described above.

The method and device for calculating a probability of having a disease according to an exemplary embodiment may contribute to identifying (backtracking) from whom the infected person got the disease.

Objects of the present invention are not limited to the above-mentioned objects, and other objects, that are not mentioned above, can be clearly understood by those skilled in the art from the following descriptions.

### [Technical Solution]

A method for calculating a probability of being infected with or having a disease according to an exemplary embodiment is disclosed. The method for calculating a probability of being infected with a disease according to an exemplary embodiment is a method carried out on a computing device with one or more processors and a memory storing one or more programs executed by the one or more processors and includes the steps of: receiving location information of each of a plurality of persons; and calculating a probability of being infected with a disease for each of the plurality of persons, on the basis of infected person information including identification information thereof and disease information thereof, and location information of each of the plurality of persons.

According to an exemplary embodiment, a device for calculating a probability of being infected with a disease is a device includes one or more processors, a memory, and one or more programs. The one or more programs are configured to be stored in the memory and executed by the one or more processors, and the program includes instructions to execute the steps of: receiving location information of each of a plurality of persons; and calculating a probability of being infected with a disease for each of the plurality of persons, on the basis of infected person information including identification information thereof and disease information thereof, and location information of each of the plurality of persons.

A method for outputting a subject to be tested for a disease according to an exemplary embodiment is a method carried out on a computing device with one or more processors and a memory storing one or more programs executed by the one or more processors and includes the steps of: receiving location information of each of a plurality of persons; receiving infected person information including identification information of an infected person and disease information of the infected person from a user; determining a subject to be tested for a disease among the plurality of persons, on the basis of the disease information of the infected person and the location information of each of the plurality of persons; and outputting information related to the subject to be tested for a disease.

A device for outputting a subject to be tested for a disease according to an exemplary embodiment is a device including one or more processors, a memory, and one or more programs. The one or more programs are configured to be stored in the memory and executed by the one or more processors, and the program includes instructions to execute the steps of: receiving location information of each of a plurality of persons; receiving infected person information including identification information of an infected person and disease information of the infected person from a user; determining a subject to be tested for a disease among the plurality of persons, on the basis of the disease information of the infected person and the location information of each of the plurality of persons; and outputting information related to the subject to be tested for a disease.

A method for calculating a probability of having a disease according to an exemplary embodiment is a method carried out on a computing device with one or more processors and a memory storing one or more programs executed by the one or more processors and includes the steps of: receiving location information of each of a plurality of persons; calculating a disease spreading probability between the plurality of persons, on the basis of infected person information including identification information thereof and disease information thereof, and location information of each of the plurality of persons; calculating a ratio of a disease spreading probability between the plurality of persons and contact persons who are in contact with the plurality of persons, with respect to each of the plurality of persons; and calculating a probability of having a disease of each of the plurality of persons, on the basis of a ratio of the disease spreading probability between the plurality of persons and contact persons who are in contact with the plurality of persons.

A device for calculating a probability of having a disease according to an exemplary embodiment is a device including one or more processors, a memory, and one or more programs. The one or more programs are configured to be stored in the memory and executed by the one or more processors, and the program includes instructions to execute the steps of: receiving location information of each of a plurality of persons; calculating a disease spreading probability between the plurality of persons, on the basis of infected person information including identification information thereof and disease information thereof, and location information of each of the plurality of persons; calculating a ratio of a disease spreading probability between the plurality of persons and contact persons who are in contact with the plurality of persons, with respect to each of the plurality of persons; and calculating a probability of having a disease of each of the plurality of persons, on the basis of a ratio of the disease spreading probability between the plurality of persons and contact persons who are in contact with the plurality of persons.

### [Advantageous Effects]

According to the exemplary embodiment, it is possible to calculate the probability of being infected with a disease of each of the plurality of persons. Further, the probability of being infected with a disease of each of the plurality of persons may be used as data to determine a subject to be tested for a disease. Specifically, the probability of being infected with a disease of each of the plurality of persons is a specific value expressed by a numerical value. Thus, it can contribute to accurately identifying the subject to be tested for a disease rather than determining only based on formal contact between the plurality of persons.

According to the exemplary embodiment, the probability of being infected with a disease of each of the plurality of persons is calculated on the basis of a disease spreading probability between the plurality of persons to contribute to more accurately identifying the subject to be tested for a disease.

According to the exemplary embodiment, the disease spreading probability between the plurality of persons is calculated on the basis of the disease spreading force and the contact degree between the plurality of persons. Thus, it can contribute to accurately identifying the subject to be tested for a disease rather than the determining on the basis of the formal contact between the plurality of persons.

According to the exemplary embodiment, the probability of being infected with a disease of a secondary contact person is calculated on the basis of the probability of being infected with a disease of a primary contact person and a disease spreading probability between the primary contact person and the second contact person. It can contribute to accurately calculating the probability of being infected with a disease of indirect contact persons as well as the person who directly contacts with the infected people.

According to the exemplary embodiment, a maximum value of the probability of being infected with a disease of a specific person calculated according to each of a plurality of contact routes is determined as a probability of being infected with a disease of the specific person. It can contribute to quickly identifying a subject to be tested for a disease by excluding minor errors.

According to an exemplary embodiment, an arithmetic mean value of the disease infection probability of each of the plurality of people may be determined as the disease infection probability of each of the plurality of people. Such an arithmetic mean value may be independently calculated by considering of the disease spreading effect by each of the plurality of infected persons. Thus, it can contribute to accurately identifying the subject to be tested for a disease by uniformly considering the effects of the plurality of infected persons.

According to the exemplary embodiment, information regarding the subject to be tested for a disease is output to help the user to more conveniently determine the subject to be tested for a disease.

According to the exemplary embodiment, a subject to be tested for a disease among the plurality of persons is determined on the basis of location information of each of the plurality of persons corresponding to a searching period. It can contribute to quickly and accurately determining the subject to be tested for a disease.

According to the exemplary embodiment, it is possible to calculate the probability of having a disease of each of the plurality of persons. Further, the probability of having a disease of each of the plurality of persons may contribute to identifying (backtracking) from whom the infected person is infected with a disease. Further, the probability of having a disease of each of the plurality of persons may be used as data to determine a person who is estimated to have a disease.

Specifically, the probability of having a disease of each of the plurality of persons is a specific value expressed by a numerical value. Thus, it can contribute to accurately identifying the person who is estimated to have a disease rather than determining only based on a formal contact between the plurality of persons.

According to the exemplary embodiment, the probability of having a disease of each of the plurality of persons is calculated on the basis of a disease spreading probability between the plurality of persons. It can contribute to more accurately identifying a person who is estimated to have a disease.

According to the exemplary embodiment, the disease spreading probability between the plurality of persons is calculated on the basis of the disease spreading power and the contact level between the plurality of persons. It can contribute accurately identifying a person who is estimated to have a disease rather than determining on the basis of the formal contact between the plurality of persons.

According to the exemplary embodiment, the probability of having a disease of a secondary contact person is calculated on the basis of the probability of having a disease of a primary contact person and a disease spreading probability between the primary contact person and the second contact person. It can contribute to accurately calculating the probability of having a disease of the indirect contact persons in addition to the person who directly contacts with the infected person.

According to the exemplary embodiment, a maximum value of the probability of having a disease of a specific person calculated according to each of a plurality of contact routes is determined as a probability of having a disease of the specific person. It can contribute to quickly identifying a person who is estimated to have a disease by excluding minor errors.

According to an exemplary embodiment, an arithmetic mean value of the probability of having a disease of each of the plurality of persons may be determined as a probability of having a disease of each of the plurality of persons. Such and arithmetic mean value may be independently calculated by considering of the disease spreading effect by each of the plurality of infected persons. Thus, it can contribute to accurately identifying the person who is estimated to have a disease by uniformly considering the effects of all of the plurality of infected persons.

### [Description of Drawings]

FIGS. 1 to 3 are exemplary diagrams for explaining a location information collecting system according to an exemplary embodiment.
FIG. 4 is a flowchart of a method for calculating a probability of being infected with a disease according to an exemplary embodiment.
FIG. 5 is a flowchart of a method for determining a subject to be tested for a disease according to an exemplary embodiment.
FIG. 6 is a flowchart of a method for calculating a probability of being infected with a disease according to another exemplary embodiment.
FIG. 7 is an exemplary view of a method for determining a subject to be tested for a disease according to another exemplary embodiment.
FIGS. 8 to 17 are exemplary views for explaining a process of calculating a probability of being infected with a disease according to an exemplary embodiment.
FIG. 18 is an exemplary view for explaining a process of determining a subject to be tested for a disease according to another exemplary embodiment.
FIG. 19 is a flowchart of a method for calculating a probability of having a disease according to an exemplary embodiment.
FIGS. 20 to 36 are exemplary views for explaining a process of calculating a probability of having a disease according to an exemplary embodiment.
FIG. 37 is an exemplary view for explaining a process of determining a person who is estimated to have a disease according to another exemplary embodiment.
FIG. 38 is a block diagram for explaining a computing environment including a computing device suitable to be used in exemplary embodiments.

### [Modes of the Invention]

Advantages and characteristics of the present invention and a method for achieving the advantages and characteristics will be clear by referring to exemplary embodiments described below in detail together with the accompanying drawings. However, the present invention is not limited to the following embodiments but may be implemented in various different forms. The embodiments are provided only to complete the disclosure of the present invention and to fully provide a person having ordinary skill in the art to which the present invention pertains with the category of the disclosure, and the present invention will be defined by the appended claims.

The shapes, sizes, ratios, angles, numbers, and the like illustrated in the accompanying drawings for describing the exemplary embodiments of the present disclosure are merely examples, and the present disclosure is not limited thereto. Further, in the following description, a detailed explanation of known related technologies may be omitted to avoid unnecessarily obscuring the subject matter of the present disclosure. The terms such as "including," "having," and "consist of' used herein are generally intended to allow other components to be added unless the terms are used with the term "only". Any references to singular may include plural unless expressly stated otherwise.

Components are interpreted to include an ordinary error range even if not expressly stated.

Although the terms "first", "second", and the like are used for describing various components, these components are not confined by these terms. These terms are merely used for distinguishing one component from the other components. Therefore, a first component to be mentioned below may be a second component in a technical concept of the present disclosure.

Like reference numerals generally denote like elements throughout the specification.

A size and a thickness of each component illustrated in the drawing are illustrated for convenience of description, and the present disclosure is not limited to the size and the thickness of the component illustrated.

The features of various embodiments of the present invention can be partially or entirely bonded to or combined with each other and can be interlocked and operated in technically various ways understood by those skilled in the art, and the embodiments can be carried out independently of or in association with each other.

In the illustrated flowcharts, even though the method is described to be divided into a plurality of steps, at least some steps are performed in a different order, or combined with another step to be performed together, omitted, or divided into sub steps, or performed by adding one or more steps which are not illustrated.

Hereinafter, various exemplary embodiments of the present invention will be described in detail with reference to accompanying drawings.

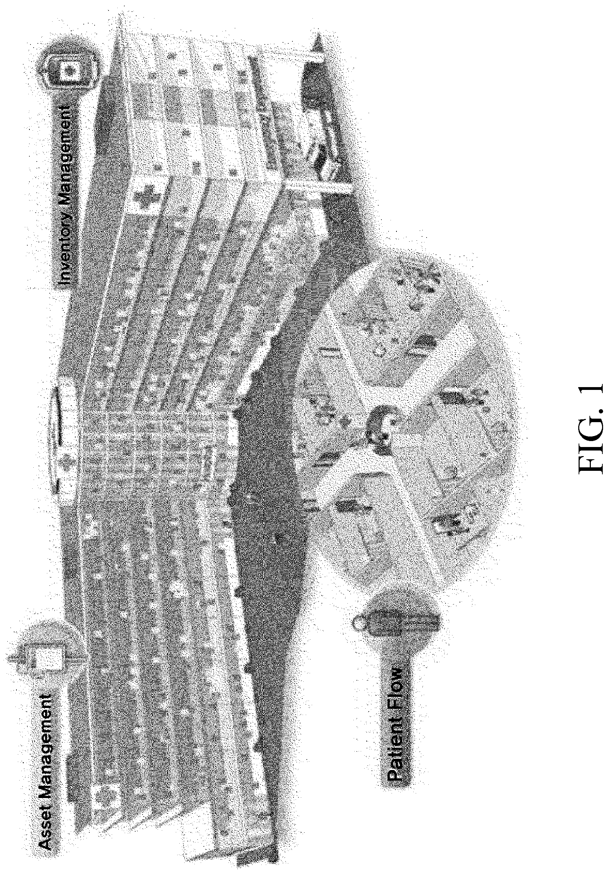

FIGS. 1 to 3 are exemplary diagrams for explaining a location information collecting system according to an exemplary embodiment.

A location information collecting system collects information about a location of a person or a device located in one space. Here, one space may be a building, a park, a sea, a coast, or the like.

In FIG. 1, a hospital is illustrated. The hospital is configured by a plurality of floors and persons and devices are located on each floor. The location information collecting system collects information about a location of a person or a device located in the hospital.

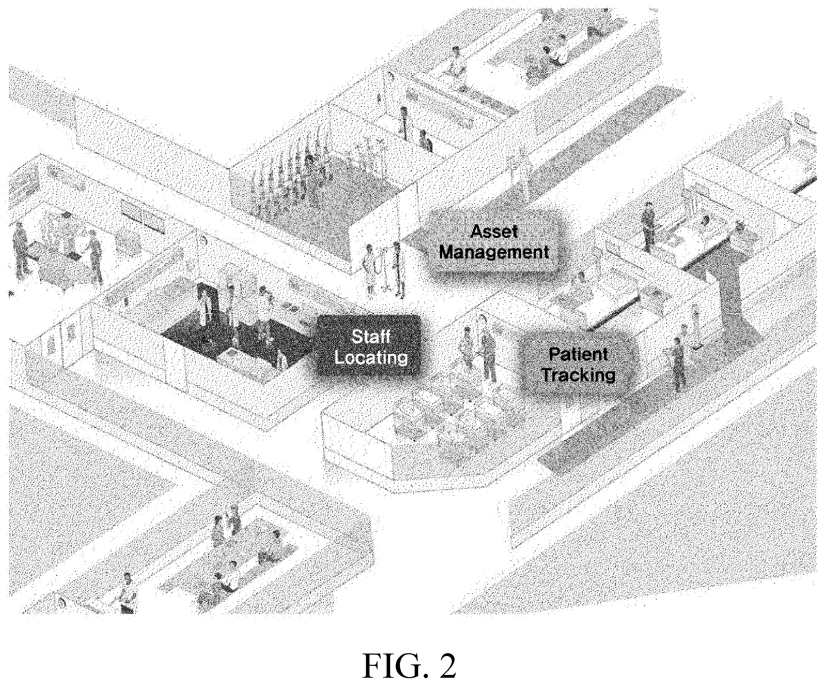

FIG. 2 illustrates a part of the inside of the hospital illustrated in FIG. 1. As illustrated in FIG. 2, the hospital includes a plurality of spaces. For example, the hospital includes staff spaces, patient spaces, device spaces, operating rooms, treatment rooms, doctor's offices, wards, restrooms, rest areas, dining rooms, hallways, and lobbies. However, it is not limited thereto and the hospital is also understood as a concept including the outside of the hospital, such as surrounding trails or gardens. In the meantime, persons in the hospital include staffs, patients, visitors, and the like.

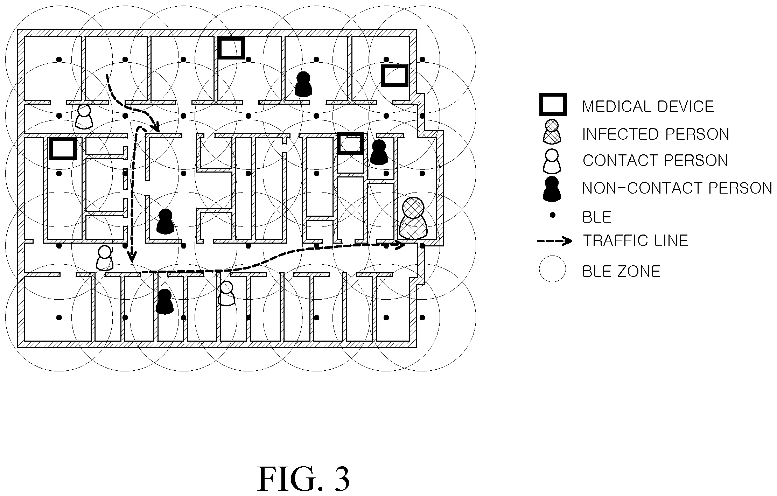

FIG. 3 visually illustrates a Bluetooth zone in a partial view of the hospital. As illustrated in FIG. 3, Bluetooth devices may be installed in the hospital. The Bluetooth devices may collect location information of persons located in the hospital. The location information collecting system may collect the location information of all the persons in the hospital by means of the Bluetooth devices. However, the location information collecting method is not limited thereto and the location information collecting system may collect the location information of the persons also using various sensors, a different IoT technique, or other communication techniques.

In the meantime, such a system is operated in such a way that persons who enter and leave the hospital hold, attach, or possess a location information transmitting device. The location information transmitting device transmits identification information or location information to the Bluetooth device in real time or at a predetermined time interval. A user maps identification information of a person who holds, attaches, or possesses the transmitting device with identification information of the transmitting device to reflect, store, and update the information in the location information collecting system. By doing this, the location information collecting system may collect the location information of all the persons in the hospital in real time or at a predetermined time interval.

Further, the location information collecting system may identify locations of persons in the hospital using a previously stored drawing of the hospital. For example, the location information collecting system maps the location corresponding to the space in the hospital, for example, staff spaces, patient spaces, device spaces, operating rooms, treatment rooms, doctor's offices, wards, restrooms, hallways, lobbies, and the like, on the drawing of the hospital. The location information collecting system identifies the locations of the persons in the hospital thereby. Further, the location information collecting system visually displays the locations of the persons on the previously stored drawing of the hospital.

Further, the location information collecting system may also store the location information of the devices in the hospital. The location information collecting system may visually display the locations of the devices on the drawing of the hospital. When the locations of the devices are changed, the location information collecting system is updated by the user to be reflected on the drawing of the hospital. Further, the devices in the hospital transmit location information thoseof to Bluetooth devices in real time, at a predetermined time interval, or when a predetermined event is generated. By doing this, the location information collecting system may also collect the location information of the devices in real time.

In the meantime, the location information collecting system generates log data of the locations of the persons at a predetermined time interval or in real time, using location information received from the location information transmitting device. The location information collecting system may identify a traffic line of a desired person during a desired time using the log data. For example, the location information collecting system may identify what time, minute, and second a specific patient is in which room or whether the patient goes to the bathroom between 1 and 2 o'clock using the log data. Further, the location information collecting system identifies how many seconds a patent A and a patient B are located to be close (for example, located within a radius of 1 m), how many minutes the patient A and the patient B are located in the specific space (for example, a rest area) together, and whether the patient A and the patient B use medical equipment X together using the log data.

By doing this, the persons in the hospital are divided into persons infected with a disease who are spreadable (hereinafter, infected person) and persons who are not infected (hereinafter, non-infected person). The location information collecting system checks whether or there is a contact capable of transmitting the disease between persons using the location information of the persons. The presence or absence of the contact may contribute to identifying whether the non-infected person is infected with a disease from the infected person.

Further, it is possible to back-track who (a person who is estimated to have a disease) spreads the disease to a person who is infected with the disease (hereinafter, an infected person) among the persons in the hospital. When the person who is infected with a disease is infected with a disease in the hospital, rather than from the outside of the hospital, the person infected with the disease may be transmitted to the disease from somebody in the hospital. The location information collecting system checks whether contact causes the spread of the disease between persons using the location information of the persons and contributes to identifying a person who is estimated to have a disease who causes the infected person to be infected with a disease on the basis of the contact.

In the meantime, the technical spirit related to the location information collecting system described in FIGS. 1 to 3 may be referenced to understand the following exemplary embodiments.

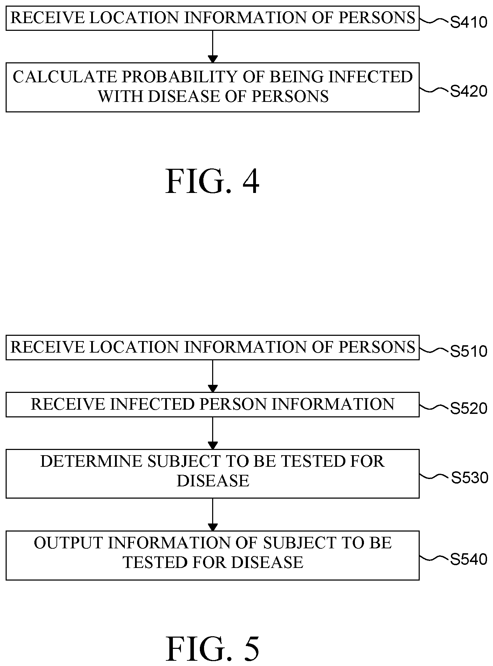

FIG. 4 is a flowchart of a method for calculating a probability of being infected with a disease according to an exemplary embodiment.

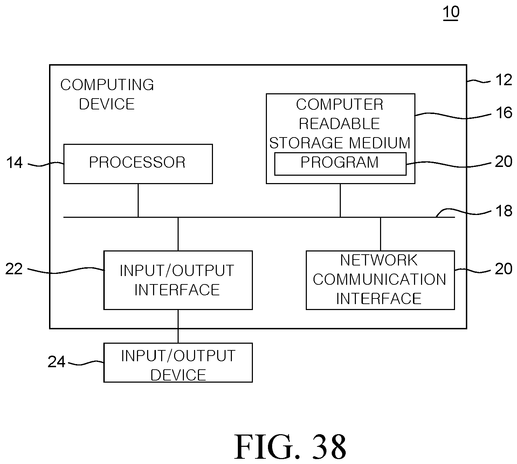

The method illustrated in FIG. 4, for example, may be performed by a computing device 12 illustrated in FIG. 38.

First, a computing device 12 receives location information of a plurality of persons (S410).

The plurality of persons, for example, may be located in the hospital described in FIGS. 1 to 3. The plurality of persons includes an infected person and a non-infected person.

For example, the computing device 12 may receive location information of the plurality of persons from Bluetooth devices located in the hospital, in real time or at a predetermined time interval.

Next, the computing device 12 calculates a probability of being infected with a disease of a plurality of persons based on infected person information and location information of each of the plurality of persons (S420).

The infected person information may include identification information of the infected person and disease information of the infected person. The information of the infected person is determined by the input of the user. For example, the computing device 12 may receive the information of the infected person from the user. Specifically, the user may input information of the infected person including identification information (for example, Hong, Gildong) of the infected person and disease information (for example, MERS) of the infected person to the computing device 12. However, the present invention is not limited thereto and the computing device 12 receives the information of the infected person from the user in such a way that an external device receives information of the infected person from the user, and the external device transmits the information of the infected person input from the user to the computing device 12. In the meantime, the information of the infected person input by the user may be information of a person who is confirmed to be infected as an infected person.

The computing device 12 may determine a disease spreading power and a risk function corresponding to the disease information of the infected person. The disease spreading power may be a value obtained by expressing a basic aggressiveness of a disease with a numerical value. The computing device 12 may store a disease spreading power set for every disease. For example, a disease A has a disease spreading power of 0.8, a disease B has a disease spreading power of 0.6, and a disease C has a disease spreading power of 0.7. For example, the user sets the disease spreading power for every disease by reflecting the aggressiveness of each disease, on the basis of the information from the Korea Disease Control and Prevention Agency or the Korean Society of Infectious Diseases. The computing device 12 may determine a disease spreading power corresponding to the disease information of the infected person using a previously stored disease spreading power for every disease.

The computing device 12 may calculate a contact degree between the plurality of persons on the basis of the risk function and the location information of each of the plurality of persons. Here, the contact degree refers to a degree of contact that can spread the disease. For example, the risk function may be a function for expressing and outputting the degree of the contact that may spread the disease between the plurality of persons with a numerical value based on the location information of the plurality of persons. Specifically, the risk function may be a weight function created by reflecting environmental factors that increase a disease spreading probability other than the disease spreading power.

For example, the risk function may express and output a degree of contact that may spread the disease between the patient A and the patient B on the basis of how many seconds the patient A and the patient B are proximately located (for example, located within a radius of 1 m), how many minutes the patient A and the patient B are located together in a specific space (for example, a rehabilitation room or the same compartment of the restroom), and whether the patient A and the patient B use the same medical device, using the location information of the patient A and the patient B. By this manner, the risk function may express the following degree numerically and output it using the location information of the patient A, the patient B, the patient C, and the patient D. For example, the risk function may output a degree of contact that may spread the disease between the patient A and the patient B, a degree of contact that may spread the disease between the patient B and a patient C, and a degree of contact that may spread the disease between the patient C and a patient D.

In the meantime, the risk function may differently set a consideration element according to a spreading route for every disease. For example, the user may differently set a consideration element of the risk function depending on whether the disease spreading route is 1) contact, 2) droplets, or 3) airborne. Specifically, the risk function for the contagious disease may express and output the degree of the contact that may spread the disease in consideration of a number and frequency of proximity within a predetermined distance, whether to use the same space (for example, the same ward, the same hallway, the same floor) and whether to share the restroom, with a numerical value. The risk function for the droplet/airborne disease may express and output the degree of contact that is capable of spreading the disease with a numerical value, in consideration of a location proximity, a type of air-conditioning device (for example, a negative pressure facility or a general ventilation) or whether there is a specific ward (for example, a clean room or a ward where a mask needs to be worn).

In this way, the computing device 12 may store a risk function set for every disease. The computing device 12 may determine a risk function corresponding to the disease information of the infected person using a previously stored risk function for every disease. For example, the patient A and the patient B were proximately located for five minutes and the patient A and the patient B were located in the same room (with a negative pressure facility) for three hours. At this time, when the contact disease becomes a problem, the risk function outputs 0.7 and when the droplet/airborne disease becomes a problem, the risk function outputs 0.5. However, this is just an example and the output value may vary depending on a detailed design of the risk function. In the meantime, the detailed matter of the risk function may be freely designed by the general technician according to a specific disease spreading route and a program policy.

Thereafter, the computing device 12 may calculate a disease spreading probability on the basis of a disease spreading power and a contact degree between the plurality of persons. For example, the computing device 12 may determine a product of the disease spreading power and a contact degree between the patient A and the patient B as a disease spreading probability between the patient A and the patient B. Thereafter, the computing device 12 may calculate a probability of being infected with a disease of each of the plurality of persons on the basis of the disease spreading probability between the plurality of persons. For example, the computing device 12 may determine a product of the probability of being infected with a disease of the patient A and a disease spreading probability between the patient A and the patient B as a probability of being infected with a disease of B. However, the disease spreading probability between the plurality of persons and the probability of being infected with a disease of each of the plurality of persons is not necessarily a probability value, but may be a value obtained by scaling the probability size. However, this is just an embodiment, and the general technician may use the technical spirit inherent in the disclosed embodiment in various and efficient manners by referring to various embodiment to be disclosed below.

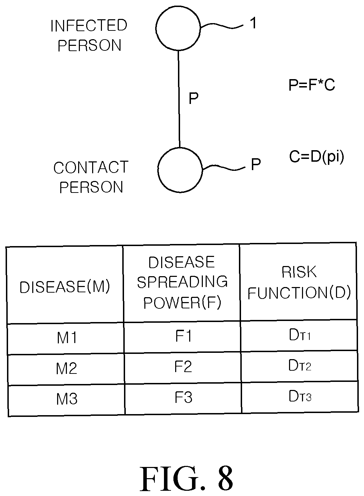

See FIG. 8. In FIG. 8, an infected person and a contact person who is in contact with the infected person are illustrated. However, this is a premise for the convenience of description and whether the infected person and the contact person are in contact with each other may be actually determined by a risk function. The computing device 12 may determine a disease spreading power F and a risk function D corresponding to the disease information of the infected person.

For example, when the disease of the infected person is M1, the computing device 12 may determine the disease spreading power as F1 and the risk function as D_{T1}. When the disease of the infected person is M2. the computing device 12 may determine the disease spreading power as F2 and the risk function as D_{T2}. When the disease of the infected person is M3, the computing device 12 may determine the disease spreading power as F3 and the risk function as D_{T3}.

The computing device 12 may calculate the contact degree C between the infected person and the contact person on the basis of the risk function D and the location information Pi of each of the infected person and the contact person. The computing device 12 may determine the product of the disease spreading power F and the contact degree C between the infected person and the contact person as a disease spreading probability P between the infected person and the contact person.

The infected person is determined by the user so that it is considered that the probability of being infected with a disease of the infected person is 1 under the premise that the infected person is an infected confirmed case. The computing device 12 may determine the product of the probability (1) of being infected with a disease of an infected person and a disease spreading probability P of the infected person and the contact person as a probability of being infected with a disease of the contact person P. In the meantime, the probability P of being infected with a disease of a contact person who is in contact with the infected person is equal to the disease spreading probability P of the infected person and the contact person. This is because the probability of being infected with a disease of the infected person is considered to be 1 under the premise that the infected person is an infected confirmed case. Accordingly, the probability of being infected with a disease of the contact person who is in contact with the infected person is considered as a disease spreading probability of the infected person and the contact person.

In the meantime, even though FIG. 8 is illustrated with an example that one contact person is in contact with the infected person. This principle may be applied in the same way as in the case that a plurality of contact persons is in contact with the infected person.

When there are a primary contact person who is in contact with the infected person and a secondary contact person who is in contact with the primary contact person, the computing device 12 may calculate a probability of being infected with a disease of the primary contact person. The computing device 12 may calculate a probability of being infected with a disease of the secondary contact person on the basis of the probability of being infected with a disease of the primary contact person and a disease spreading probability between the primary contact person and the secondary contact person. For example, the computing device 12 may determine the product of the probability of being infected with a disease of the primary contact person, and the disease spreading probability between the primary contact person and the secondary contact person as a probability of being infected with a disease of the secondary contact person.

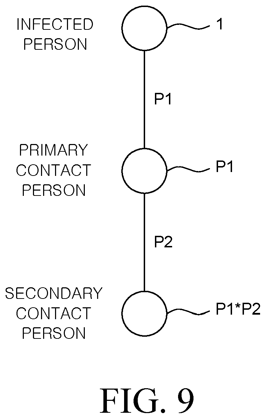

See FIG. 9. In FIG. 9, the primary contact person who is in contact with the infected person and the secondary contact person who is in contact with the primary contact person are illustrated. This is a premise for the convenience of description and whether the infected person is in contact with the primary contact person and whether the primary contact person is in contact with the secondary contact person may be actually determined by the risk function.

The computing device 12 may calculate the probability of being infected with a disease of the primary contact person who is in contact with the infected person by the method described with reference to FIG. 8. For example, the computing device 12 may determine the disease spreading probability P1 of the primary contact person who is in contact with the infected person as a probability P1 of being infected with a disease of the primary contact person.

For example, the computing device 12 may determine the product of the probability P1 of being infected with a disease of the primary contact person, and the disease spreading probability P2 between the primary contact person and the secondary contact person as a probability P1^{∗}P2 of being infected with a disease of the secondary contact person. The probability P1^{∗}P2 of being infected with a disease of the secondary contact person is equal to a product of the disease spreading probability P2 between the infected person and the primary contact person, and the disease spreading probability P2 between the primary contact person and the secondary contact person. This is because the probability of being infected with a disease of the infected person is considered to be 1 under the premise that the infected person is an infected confirmed case. Accordingly, the probability P1^{∗}P2 of being infected with a disease of the secondary contact person is considered as a product of the disease spreading probability P2 between the infected person and the primary contact person, and the disease spreading probability P2 between the primary contact person and the secondary contact person.

In the meantime, even though FIG. 9 is described with an example that there is only the primary and secondary contact persons. This principle is expanded to the case where there are a tertiary contact person who is in contact with the secondary contact person, a quaternary contact person who is in contact with the tertiary contact person, ..., and an n-th order contact person. For example, when a disease spreading probability of the tertiary contact person who is in contact with the secondary contact person is P3, a probability of being infected with a disease of the tertiary contact person is P1^{∗}P2^{∗}P3. When a disease spreading probability of the quaternary contact person who is in contact with the tertiary contact person is P4, a probability of being infected with a disease of the quaternary contact person is P1^{∗}P2^{∗}P3^{∗}P4. Further, a probability of being infected with a disease of the n-th order contact person is P1^{∗}P2^{∗}P3^{∗}P4^{∗}P5.

When there is a plurality of contact routes between the infected person and a specific person, the computing device 12 may calculate a probability of being infected with a disease of the specific person according to each of the plurality of contact routes. The computing device 12 may determine the maximum value of the probability of being infected with a disease of the specific person calculated according to each of the plurality of contact routes as the probability of being infected with a disease of the specific person.

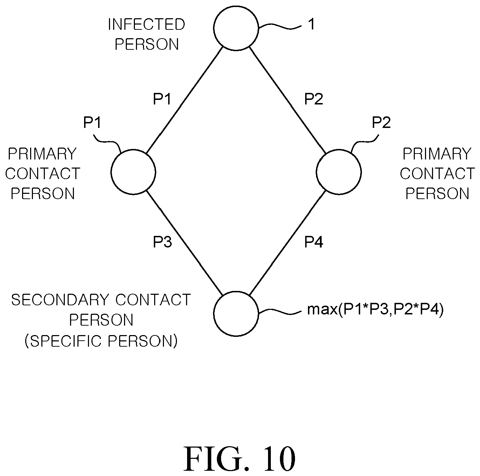

See FIG. 10. In FIG. 10, two primary contact persons who are in contact with the infected person and a secondary contact person (specific person) who is in contact with two primary contact persons are illustrated. This is a premise for the convenience of description and whether the infected person is in contact with two primary contact persons and whether two primary contact persons are in contact with the specific person may be actually determined by the risk function.

Referring to FIG. 10, there are two contact routes between the infected person and the specific person.

The computing device 12 may calculate a probability of being infected with a disease of the specific person according to the contact route that passes through the primary contact person at the left side of the drawing by the method described with reference to FIG. 9. The computing device 12 may calculate a probability of being infected with a disease of the specific person according to the contact route that passes through the primary contact person at the right side of the drawing by the method described with reference to FIG. 9. For example, the computing device 12 may determine the probability of being infected with a disease of the specific person according to the contact route via the primary contact person at the left side on the drawing as P1^{∗}P3. Further, the computing device 12 may determine the probability of being infected with a disease of the specific person according to the contact route via the primary contact person at the right side on the drawing as P2^{∗}P4.

At this time, the computing device 12 may determine the maximum value {max(P1^{∗}P3, P2^{∗}P4)} as a probability of being infected with a disease of the specific person. That is, the computing device 12 may select the maximum value of the probability P1^{∗}P3 of being infected with a disease of the specific person calculated according to the contact route via the primary contact person at the left side on the drawing and the probability P2^{∗}P4 of being infected with a disease of the specific person according to the contact route via the primary contact person at the right side on the drawing.

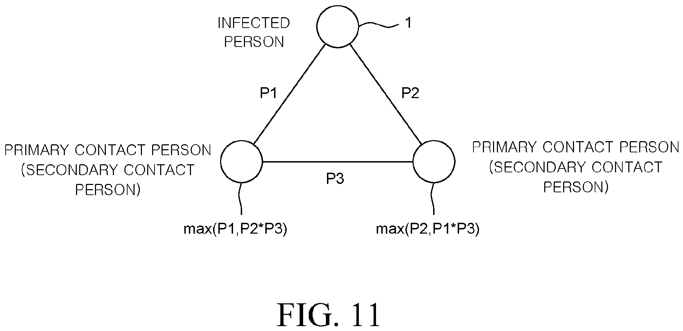

See FIG. 11. In FIG. 11, two primary contact persons who are in contact with the infected person are illustrated. Further, a primary contact person at the left side on the drawing has been in contact with a primary contact person at the right side on the drawing. This is a premise for the convenience of description and whether the infected person is in contact with two primary contact persons and whether two primary contact persons are in contact with each other may be actually determined by the risk function.

Referring to FIG. 11, there are two contact routes between the infected person and each of the primary contact persons. In the meantime, the primary contact person at the left side on the drawing has been in direct contact with the infected person and has been in indirect contact with the infected person via the primary contact person at the right side on the drawing. Therefore, the primary contact person at the left side on the drawing simultaneously becomes a primary contact person and a secondary contact person. Further, the primary contact person at the right side on the drawing has been in direct contact with the infected person and has been in indirect contact with the infected person via the primary contact person at the left side on the drawing. Therefore, the primary contact person at the right side on the drawing simultaneously becomes a primary contact person and a secondary contact person.

The computing device 12 may determine the probability of being infected with a disease of the primary contact person at the left side on the drawing, according to a direct contact route with the infected person as P1, by the method described with reference to FIG. 8. Further, the computing device 12 may determine the probability of being infected with a disease of the primary contact person at the right side on the drawing, according to a direct contact route with the infected person as P2, by the method described with reference to FIG. 8.

Further, the computing device 12 may determine the probability of being infected with a disease of the primary contact person at the left side on the drawing according to the contact route via the primary contact person at the right side on the drawing as P2^{∗}P3, by the method described with reference to FIG. 9. Further, the computing device 12 may determine the probability of being infected with a disease of the primary contact person at the right side on the drawing according to the contact route via the primary contact person at the left side on the drawing as P1^{∗}P3, by the method described with reference to FIG. 9.

At this time, the computing device 12 may determine the maximum value {max(P1, P2^{∗}P3)} between the probability P1 of being infected with a disease of the primary contact person at the left side on the drawing and the probability P2^{∗}P3 of being infected with a disease of the primary contact person at the left side on the drawing as the probability of being infected with a disease of the primary contact person at the left side on the drawing. Further, the computing device 12 determines the maximum value {max(P1, P1^{∗}P3)} between the probability P2 of being infected with a disease of the primary contact person at the right side on the drawing and the probability P1^{∗}P3 of being infected with a disease of the primary contact person at the right side on the drawing as the probability of being infected with a disease of the primary contact person at the right side on the drawing.

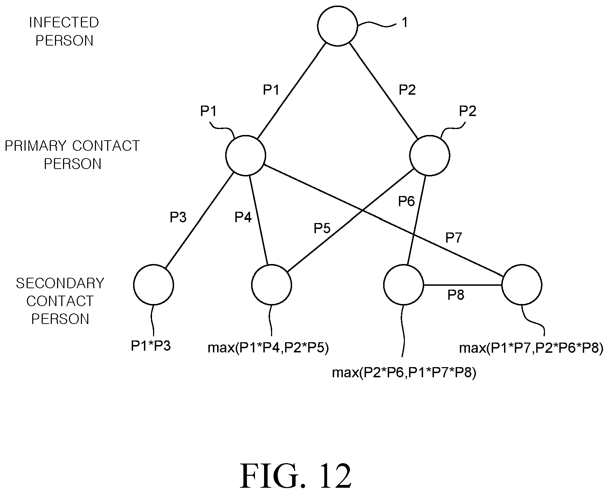

See FIG. 12. In FIG. 12, one infected person, two primary contact persons who are in contact with the infected person, and four secondary contact persons who are in contact with the primary contact person are illustrated. Further, a first secondary contact person from the right side among the secondary contact persons has been in contact with the secondary contact person at the left side thereof. This is a premise for the convenience of description and whether the infected person is in contact with two primary contact persons, whether two primary contact persons are in contact with four secondary contact persons, and whether a right first secondary contact person and the secondary contact person of the left side thereof among the secondary contact persons are in contact with each other may be determined by the risk function.

The computing device 12 may calculate a probability of being infected with a disease of each of one infected person, two primary contact persons who are in contact with the infected person, and four secondary contact persons who are in contact with the primary contact person, by the method described with reference to FIGS. 8 to 11. Since two primary contact persons are in direct contact with the infected person, probabilities of being infected with a disease of two primary contact persons may be determined as P1 and P2.

Since the secondary contact person at the left outermost side has only a contact route via the primary contact person at the left side on the drawing, the probability of being infected with a disease of the secondary contact person at the left outermost side may be determined as P1^{∗}P3. Since the second secondary contact person at the left side has a contact route via the primary contact person at the left side on the drawing and a contact route via the primary contact person at the right side on the drawing, the probability of being infected with a disease of the second secondary contact person at the left side may be determined as max (P1^{∗}P4, P2^{∗}P5).

Since a third secondary contact person at the left side has a contact route via the primary contact person at the right side on the drawing and a contact route via the primary contact person at the left side on the drawing and the first secondary contact person at the right side, a probability of being infected with a disease of a third secondary contact person at the left side may be determined as max(P2^{∗}P6, P1^{∗}P7^{∗}P8). Since a first secondary contact person at the right side has a contact route via the primary contact person at the left side on the drawing and a contact route via the primary contact person at the right side on the drawing and the second secondary contact person at the right side, a probability of being infected with a disease of a first secondary contact person at the right side may be determined as max(P1^{∗}P7, P2^{∗}P6^{∗}P8).

When there is a plurality of infected persons, the computing device 12 may independently calculate a probability of being infected with a disease of each of the plurality of persons in consideration of a disease spreading effect by each of the plurality of infected persons. The computing device 12 may determine an arithmetic mean value of the probabilities of being infected with a disease of the plurality of persons. Here, the arithmetic mean value is independently calculated in consideration of the disease spreading effect by each of the plurality of infected persons. Thus, the computing device 12 may calculate a probability of being infected with a disease of each of the plurality of persons.

For example, when two infected persons are a first infected person and a second infected person, the computing device 12 may calculate the probability of being infected with a disease of each of the plurality of persons in consideration of only the disease spreading effect by the first infected person. The computing device 12 may calculate the probability of being infected with a disease of each of the plurality of persons in consideration of only the disease spreading effect by the second infected person. And then, the computing device 12 may determine the probability of being infected with a disease of each of the plurality of persons as an arithmetic value of the two previous probabilities of being infected with a disease.

In the meantime, when the probability of being infected with a disease of each of the plurality of persons is calculated in consideration of only the disease spreading effect by the first infected person. It means that when the probability of being infected with a disease of each of the plurality of persons is calculated in consideration of only the disease spreading effect by the first infected person, there is no disease spreading effect of an infected person (the second infected person) other than the first infected person. Further, when the probability of being infected with a disease of each of the plurality of persons is calculated in consideration of only the disease spreading effect by the second infected person. It means that when the probability of being infected with a disease of each of the plurality of persons is calculated in consideration of only the disease spreading effect by the second infected person, there is no disease spreading effect of an infected person (the first infected person) other than the second infected person. By expanding this, it is possible to independently calculate the probability of being infected with a disease of each of the plurality of persons in consideration of the disease spreading effect by each of the plurality of infected persons.

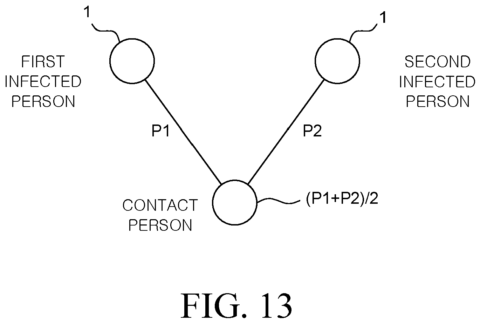

See FIG. 13. In FIG. 13, a contact person who is in contact with the first infected person and the second infected person is illustrated. This is a premise for the convenience of description and whether the first infected person is in contact with the contact person and whether the second infected person is in contact with the contact person may be determined by the risk function.

The computing device 12 may determine the probability of being infected with a disease of the contact person as P1 in consideration of only the disease spreading effect by the first infected person. Further, the computing device 12 may determine the probability of being infected with a disease of the contact person as P2 in consideration of only the disease spreading effect by the second infected person. At this time, the computing device 12 may determine the arithmetic mean {(P1+P2)/2} of the probability P1 of being infected with a disease of the contact person calculated in consideration of only the disease spreading effect by the first infected person and the probability P2 of being infected with a disease of the contact person in consideration of only the disease spreading effect by the second infected person as the probability of being infected with a disease of the contact person. By expanding this, unlike FIG. 13, there is a contact person in contact with the first infected person, the second infected person, ..., and an n-th infected person, the probability of being infected with a disease of the contact person may be {(P1+P2+...+Pn)/n}.

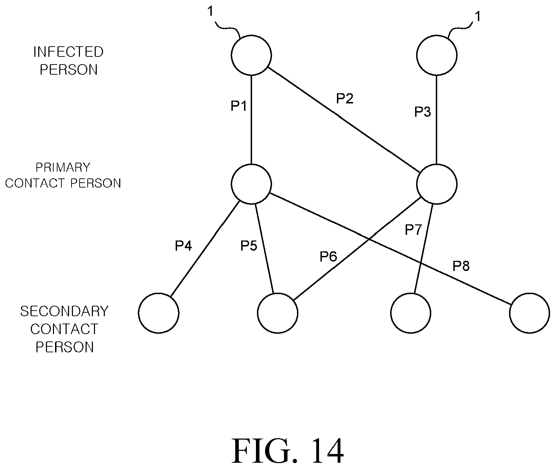

See FIG. 14. In FIG. 14, two infected persons, two primary contact persons who are in contact with two infected persons, and four secondary contact persons who are in contact with the primary contact person are illustrated. This is a premise for the convenience of description and whether two infected persons are in contact with two primary contact persons and whether two primary contact persons are in contact with four secondary contact persons may be determined by the risk function.

Since two infected persons are a first infected person and a second infected person, the computing device 12 may calculate a probability of being infected with a disease of each of the plurality of persons in consideration of only the disease spreading effect by the first infected person. The computing device 12 may calculate a probability of being infected with a disease of each of the plurality of persons in consideration of only the disease spreading effect by the second infected person. The computing device 12 may determine an arithmetic mean value of the probability of being infected with a disease of each of the plurality of persons calculated and the probability of being infected with a disease of each of the plurality of persons calculated as a probability of being infected with a disease of each of the plurality of persons.

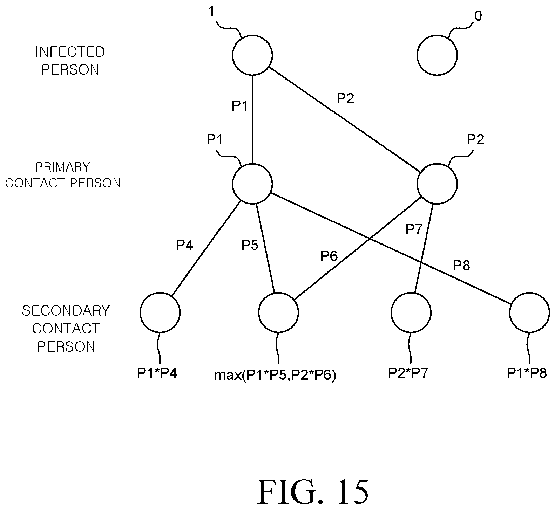

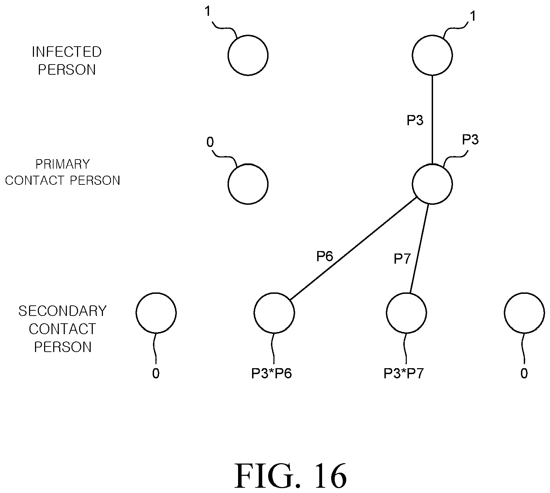

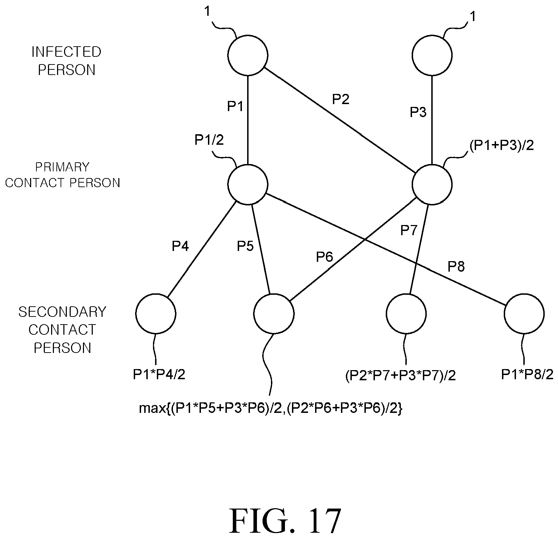

FIG. 15 illustrates a probability of being infected with a disease of each of the plurality of persons calculated in consideration of only the disease spreading effect by the first infected person in FIG. 14. At this time, the probability of being infected with a disease of the second infected person is considered to be zero so that it may be ignored. Further, FIG. 16 illustrates a probability of being infected with a disease of each of the plurality of persons calculated in consideration of only the disease spreading effect by the first infected person in FIG. 14. At this time, the probability of being infected with a disease of the first infected person is considered to be zero so that it may be ignored. FIG. 17 illustrates an arithmetic mean value of the probability of being infected with a disease of each of the plurality of persons calculated in consideration of only the disease spreading effect by the first infected person (see FIG. 15) and the probability of being infected with a disease of each of the plurality of persons calculated in consideration of only the disease spreading effect by the second infected person (see FIG. 16).

By expanding this, unlike FIGS. 14 to 17, when there are a total of n infected persons including the first infected person, the second infected person to an n-th infected person, the computing device 12 may independently calculate the probability of being infected with a disease of each of the plurality of persons in consideration of the disease spreading effect by each of the plurality of infected persons. The computing device 12 may determine the arithmetic mean value of the probability of being infected with a disease of each of the plurality of persons as a probability of being infected with a disease of each of the plurality of persons. Here, the arithmetic mean value is independently calculated in consideration of the disease spreading effect by each of the plurality of infected persons. Thus, the computing device 12 may calculate a probability of being infected with a disease of each of the plurality of persons.

By doing this, the computing device 12 may calculate the probability of being infected with a disease of each of the plurality of persons only with the infected person information input by the user. The probability of being infected with a disease of each of the plurality of persons may be used as data to determine a subject to be tested for a disease.

FIG. 5 is a flowchart of a method for determining a subject to be tested for a disease according to an exemplary embodiment.

The method illustrated in FIG. 5, for example, may be performed by a computing device 12 illustrated in FIG. 38.

The method illustrated in FIG. 4 may be applied to a method illustrated in FIG. 5 in the same or similar way within a range that is not against the property. Accordingly, the repeated description with FIG. 4 may be omitted within a range that excessively clouds the gist.

First, a computing device 12 receives location information of a plurality of persons (S510).

The plurality of persons, for example, may be located in the hospital described in FIGS. 1 to 3. The plurality of persons includes an infected person and a non-infected person.

For example, the computing device 12 may receive location information of the plurality of persons from Bluetooth devices located in the hospital, in real time or at a predetermined time interval.

Next, the computing device 12 receives information of the infected person from the user (S520). The information of the infected person may include identification information of the infected person and disease information of the infected person. For example, the computing device 12 may receive the information of the infected person from the user. Specifically, the user may input information of the infected person including identification information (for example, Hong, Gildong) of the infected person and disease information (for example, MERS) of the infected person to the computing device 12. However, the present invention is not limited thereto and the computing device 12 receives the information of the infected person from the user in such a way that an external device receives information of the infected person from the user and the external device transmits the information of the infected person input from the user to the computing device 12.

Next, the computing device 12 determines a subject to be tested for a disease among a plurality of persons on the basis of disease information of the infected person and location information of each of the plurality of persons (S530).

The computing device 12 calculates a probability of being infected with a disease of each of the plurality of persons on the basis of the information of the infected person and the location information of each of the plurality of persons. The computing device 12 determines a subject to be tested for a disease among the plurality of persons on the basis of the probability of being infected with a disease of each of the plurality of persons. Here, in order to calculate the probability of being infected with a disease of each of the plurality of persons, the method described with reference to FIG. 4 may be applied in the same or similar way.

According to an exemplary embodiment, the computing device 12 receives a searching period from the user and determines a subject to be tested for a disease among the plurality of persons on the basis of the location information of each of the plurality of persons corresponding to the searching period. For example, the computing device 12 receives the searching period from the user, calculates a probability of being infected with a disease of each of the plurality of persons on the basis of the information of the infected person and the location information of each of the plurality of persons corresponding to the searching period, and determines a subject to be tested for a disease among the plurality of persons on the basis of the probability of being infected with a disease of each of the plurality of persons. By doing this, when the subject to be tested for a disease is determined, an inflow timing of the infected person or a disease onset timing is considered to reduce an unnecessary computation amount. For example, the user inputs the inflow timing of the infected person or the disease onset timing as a starting timing of the searching period and the current timing as an end timing of the searching period.

According to the exemplary embodiment, the computing device 12 determines a disease latent period corresponding to disease information of the infected person and sets the searching period on the basis of the disease latent period. For example, the computing device 12 stores a latent period table for every disease and determines a disease latent period corresponding to the disease information of the infected person on the basis of the latent period table for every disease. Thereafter, the computing device 12 determines a subject to be tested for a disease among the plurality of persons on the basis of the location information of each of the plurality of persons corresponding to the searching period. For example, the computing device 12 receives the searching period from the user and calculates a probability of being infected with a disease of each of the plurality of persons on the basis of the information of the infected person and the location information of each of the plurality of persons corresponding to the searching period. And then, the computing device 12 determines a subject to be tested for a disease among the plurality of persons on the basis of the probability of being infected with a disease of each of the plurality of persons.

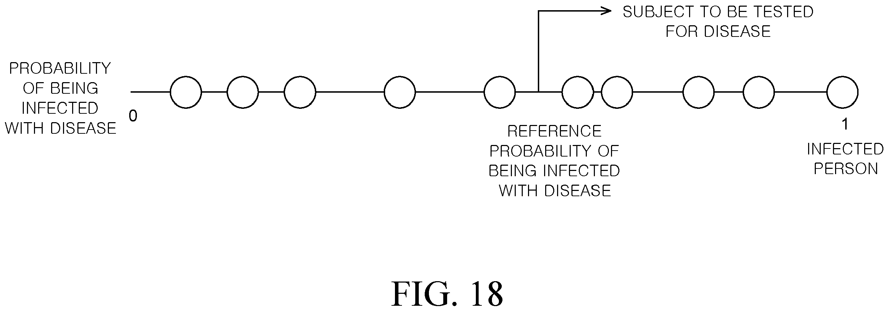

As illustrated in FIG. 18, the computing device 12 may determine a person whose probability of being infected with a disease exceeds a predetermined reference probability of being infected with a disease, among the plurality of persons, as a subject to be tested for a disease. In the meantime, the user inputs the reference probability of being infected with a disease to the computing device 12 to set the reference probability of being infected with a disease.

Next, the computing device 12 outputs information related to the subject to be tested for a disease (S540). For example, the computing device 12 may visually or acoustically output information related to the subject to be tested for a disease. For example, the computing device 12 may output identification information of the subject to be tested for a disease.

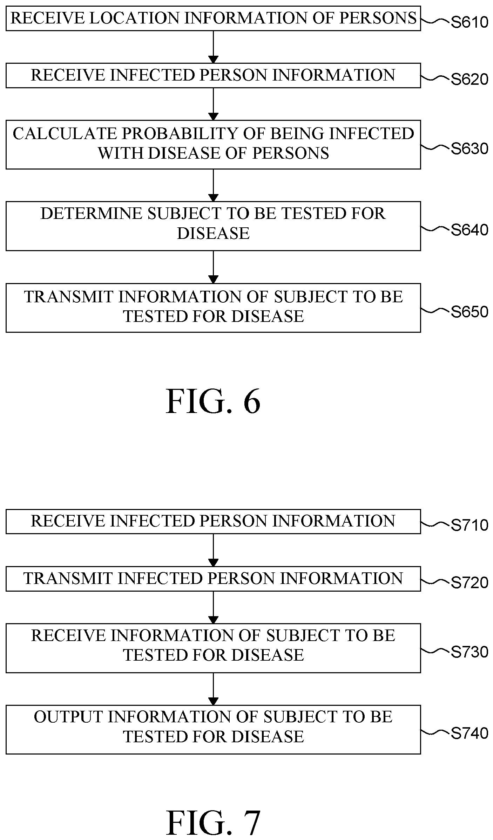

FIG. 6 is a flowchart of a method for calculating a probability of being infected with a disease according to another exemplary embodiment.

The method illustrated in FIG. 6, for example, may be performed by a computing device 12 illustrated in FIG. 38.

The method illustrated in FIGS. 4 and 5 may be applied to a method illustrated in FIG. 6 in the same or similar way within a range that is not against the property. Accordingly, the repeated description with FIGS. 4 and 5 may be omitted within a range that excessively clouds the gist.

First, a computing device 12 receives location information of a plurality of persons (S610).

The plurality of persons, for example, may be located in the hospital described in FIGS. 1 to 3. The plurality of persons includes an infected person and a non-infected person.

For example, the computing device 12 may receive location information of the plurality of persons from Bluetooth devices located in the hospital, in real time or at a predetermined time interval.

Next, the computing device 12 receives information of an infected person from an external device (S620). The information of the infected person may include identification information of the infected person and disease information of the infected person. The external device receives information of the infected person from the user and transmits the information of the infected person to the computing device 12.

Next, the computing device 12 calculates a probability of being infected with a disease of a plurality of persons based on information of the infected person and location information of each of the plurality of persons (S630). Here, in order to calculate the probability of being infected with a disease of each of the plurality of persons, the method described with reference to FIG. 4 may be applied in the same or similar way.

Next, the computing device 12 determines a subject to be tested for a disease among the plurality of persons on the basis of the probability of being infected with a disease of each of the plurality of persons (S640).

As illustrated in FIG. 18, the computing device 12 may determine a person whose probability of being infected with a disease exceeds a predetermined reference probability of being infected with a disease, among the plurality of persons, as a subject to be tested for a disease.

Next, the computing device 12 outputs information related to the subject to be tested for a disease to the external device (S650).

At this time, the external device may output information related to the subject to be tested for a disease. For example, the external device may visually, or acoustically output information related to the subject to be tested for a disease. For example, the external device may output identification information of the subject to be tested for a disease.

FIG. 7 is a flowchart of a method for determining a subject to be tested for a disease according to another exemplary embodiment.

The method illustrated in FIG. 7, for example, may be performed by a computing device 12 illustrated in FIG. 38.

The method illustrated in FIGS. 4 to 6 may be applied to a method illustrated in FIG. 7 in the same or similar way within a range that is not against the property. Accordingly, the repeated description with FIGS. 4 to 6 may be omitted within a range that excessively clouds the gist.

First, the computing device 12 receives information of the infected person from the user (S710). The information of the infected person may include identification information of the infected person and disease information of the infected person. For example, the computing device 12 may receive the information of the infected person from the user. Specifically, the user may input information of the infected person including identification information (for example, Hong, Gildong) of the infected person and disease information (for example, MERS) of the infected person to the computing device 12. However, the present invention is not limited thereto and the computing device 12 receives the information of the infected person from the user in such a way that an external device receives information of the infected person from the user and the external device transmits the information of the infected person input from the user to the computing device 12.

Next, the computing device 12 transmits information of the infected person input from the user to the external device (S720).

The external device receives location information of the plurality of persons from the Bluetooth devices in real time or at a predetermined time interval. When the external device receives the information of the infected person, the external device may calculate the probability of being infected with a disease of each of the plurality of persons on the basis of information of the infected person and location information of each of the plurality of persons. Here, in order to calculate the probability of being infected with a disease of each of the plurality of persons, the method described with reference to FIG. 4 may be applied in the same or similar way.

The external device may determine the subject to be tested for a disease among the plurality of persons on the basis of the probability of being infected with a disease of each of the plurality of persons. For example, the external device may determine a person whose probability of being infected with a disease exceeds a predetermined reference probability of being infected with a disease, among the plurality of persons, as a subject to be tested for a disease.

The external device may transmit the information related to the subject to be tested for a disease to the computing device 12.

The computing device 12 receives information related to the subject to be tested for a disease from the external device (S730).

Next, the computing device 12 outputs information related to the subject to be tested for a disease (S740). For example, the computing device 12 may visually or acoustically output information related to the subject to be tested for a disease. For example, the computing device 12 may output identification information of the subject to be tested for a disease.

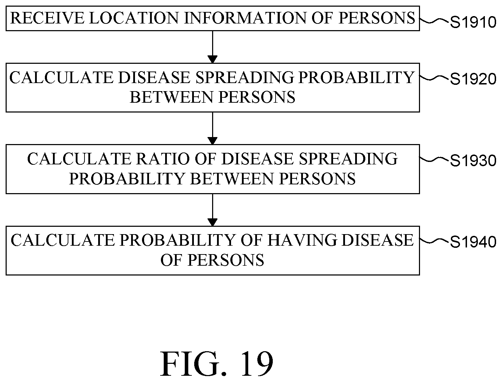

FIG. 19 is a flowchart of a method for calculating a probability of having a disease according to an exemplary embodiment.

The method illustrated in FIG. 19, for example, may be performed by a computing device 12 illustrated in FIG. 38.

First, a computing device 12 receives location information of a plurality of persons (S 1910).

The plurality of persons, for example, may be located in the hospital described in FIGS. 1 to 3. The plurality of persons includes an infected person and a non-infected person.

For example, the computing device 12 may receive location information of the plurality of persons from Bluetooth devices located in the hospital, in real time or at a predetermined time interval.

Next, the computing device 12 calculates a disease spreading probability between a plurality of persons on the basis of information of the infected person including identification information of the infected person and disease information of the infected person and location information of each of the plurality of persons (S 1920).

The information of the infected person may include identification information of the infected person and disease information of the infected person. The information of the infected person is determined by the input of the user. For example, the computing device 12 may receive the information of the infected person from the user. Specifically, the user may input information of the infected person including identification information (for example, Hong, Gildong) of the infected person and disease information (for example, MERS) of the infected person to the computing device 12. However, the present invention is not limited thereto and the computing device 12 receives the information of the infected person from the user in such a way that an external device receives information of the infected person from the user and the external device transmits the information of the infected person input from the user to the computing device 12. In the meantime, the information of the infected person input by the user may be information of a person who is confirmed to be infected as an infected person.

The computing device 12 may determine a disease spreading power and a risk function corresponding to the disease information of the infected person. The disease spreading power may be a value obtained by expressing a basic aggression of a disease with a numerical value. The computing device 12 may store a disease spreading power set for every disease. For example, a disease A has a disease spreading power of 0.8, a disease B has a disease spreading power of 0.6, and a disease C has a disease spread of 0.7. For example, the user sets the disease spreading power for every disease by reflecting the aggressiveness of each disease, on the basis of the information from the Korea Disease Control and Prevention Agency or the Korean Society of Infectious Diseases. The computing device 12 may determine a disease spreading power corresponding to the disease information of the infected person using a previously stored disease spreading power for every disease.

The computing device 12 may calculate a contact degree between the plurality of persons on the basis of the risk function and the location information of each of the plurality of persons. Here, the contact degree refers to a degree of contact that may spread the disease. For example, the risk function may be a function for expressing and outputting the degree of the contact that may spread the disease between the plurality of persons with a numerical value based on the location information of the plurality of persons. Specifically, the risk function may be a weight function created by reflecting environmental factors increasing a probability of spreading a disease other than the disease spreading power.

For example, the risk function may express and output a degree of contact that may spread the disease between the patient A and the patient B on the basis of how many seconds the patient A and the patient B are proximately located (for example, located within a radius of 1 m), how many minutes the patient A and the patient B are located together in a specific space (for example, a rehabilitation room or the same compartment of the restroom), and whether the patient A and the patient B use the same medical device, using the location information of the patient A and the patient B. By this manner, the risk function may express the following degree numerically and output it using the location information of the patient A, the patient B, the patient C, and the patient D. For example, the risk function may output a degree of contact that may spread the disease between the patient A and the patient B, a degree of contact that may spread the disease between the patient B and a patient C, and a degree of contact that may spread the disease between the patient C and a patient D.

In the meantime, the risk function may differently set a consideration element according to a spreading route for every disease. For example, the user may differently set a consideration element of the risk function depending on whether the disease spreading route is 1) contact, 2) droplets, or 3) airborne. Specifically, the risk function for the contagious disease may express and output the degree of the contact that may spread the disease in consideration of a number and frequency of proximity within a predetermined distance, whether to use the same space (for example, the same ward, the same hallway, the same floor) and whether to share the restroom with a numerical value. The risk function for the droplet/airborne disease may express and output the degree of contact that is capable of spreading the disease with a numerical value, in consideration of a location proximity, a type of air-conditioning device (for example, a negative pressure facility or a general ventilation) or whether there is a specific ward (for example, a clean room or a ward where a mask needs to be worn).

In this way, the computing device 12 may store a risk function set for every disease. The computing device 12 may determine a risk function corresponding to the disease information of the infected person using a previously stored risk function for every disease. For example, the patient A and the patient B were proximately located for five minutes and the patient A and the patient B were located in the same room (with a negative pressure facility) for three hours. At this time, when the contact disease becomes a problem, the risk function outputs 0.7 and when the droplet/airborne disease becomes a problem, the risk function outputs 0.5. However, this is just an example and the output value may vary depending on a detailed design of the risk function. In the meantime, the detailed matter of the risk function may be freely designed by the general technician according to a specific disease spreading route and a program policy.

Thereafter, the computing device 12 may calculate a disease spreading probability on the basis of a disease spreading power and a contact degree between the plurality of persons. For example, the computing device 12 may determine a product of the disease spreading power and a contact degree between the patient A and the patient B as a disease spreading probability between the patient A and the patient B.

The computing device 12 may calculate a ratio of a disease spreading probability between a plurality of persons and contact persons who are in contact with the plurality of persons with respect to each of the plurality of persons (S1930). Details will be described below with reference to FIGS. 5 to 21.

Thereafter, the computing device 12 may calculate a probability of being infected with a disease of each of the plurality of persons on the basis of the ratio of a disease spreading probability between a plurality of persons and contact persons who are in contact with the plurality of persons (S 1940). For example, the computing device 12 may determine a product of the probability of having a disease of the patient A and a ratio of a disease spreading probability of the patient A with respect to the patient B as a probability of having a disease of the patient B. However, the disease spreading probability between the plurality of persons and the probability of having a disease of each of the plurality of persons is not necessarily a probability value, but may be a value obtained by scaling the probability size. However, this is just an embodiment and the general technician may use the technical spirit inherent in the disclosed embodiment in various and efficient manners by referring various embodiments to be disclosed below.

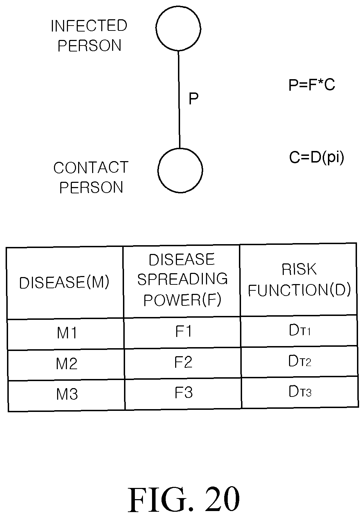

See FIG. 20. In FIG. 20, an infected person and a contacted person who is in contact with the infected person are illustrated. However, this is a premise for the convenience of description and whether the infected person and the contact person are in contact may be actually determined by a risk function. The computing device 12 may determine a disease spreading power F and a risk function D corresponding to the disease information of the infected person.

For example, when the disease of the infected person is M1, the computing device 12 may determine the disease spreading power as F1 and the risk function as D_{T1}.When the disease of the infected person is M2, the computing device 12 may determine the disease spreading power as F2 and the risk function as D_{T2}. When the disease of the infected person is M3, the computing device 12 may determine the disease spreading power as F3 and the risk function as D_{T3}.

The computing device 12 calculates the contact degree C between the infected person and the contact person on the basis of the risk function D and the location information Pi of each of the infected person and the contact person. The computing device 12 determines the product of the disease spreading power F and the contact degree C between the infected person and the contact person as a disease spreading probability P between the infected person and the contact person.

In the meantime, when there is a plurality of contact persons who are in contact with a specific person among the plurality of persons, a ratio of a disease spreading probability of a specific person for a specific contact person among a plurality of contact persons may be calculated on the basis of a value obtained by dividing a disease spreading probability between the specific person and the specific contact person by a sum of a disease spreading probability between the specific person and the plurality of contact persons. For example, when the patient A is in contact with a plurality of patients, a ratio of the disease spreading probability may refer to a probability of spreading a disease to the patient A from a patient B among the plurality of patients. Specifically, the ratio of the disease spreading probability of the patient A for the patient B refers to a probability of spreading a disease to the patient A from the patient B among the plurality of patients.

In the meantime, when a contact person who is in contact with a specific person among a plurality of persons is a single specific contact person, a ratio of a disease spreading probability of a specific person for a specific contact person among a plurality of contact persons may be calculated on the basis of a value obtained by dividing a disease spreading probability between the specific person and the specific contact person by a disease spreading probability between the specific person and the specific contact person. For example, when a contact person who is in contact with a specific person among a plurality of persons is a single specific contact person, a ratio of a disease spreading probability of the specific person for the specific contact person may be 1. This is because when a contact person who is in contact with a specific person among a plurality of persons is a single specific contact person, if the specific person is an infected person, the specific person may be infected with a disease from the specific contact person.

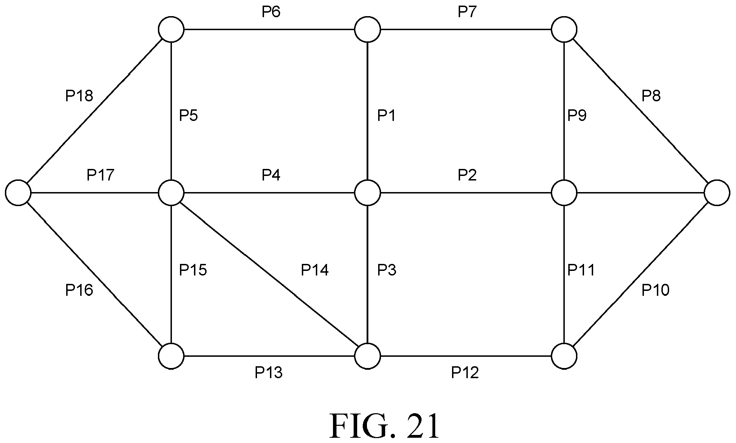

See FIG. 21. FIG. 21 illustrates a contact relationship of a plurality of persons and a disease spreading probability between the plurality of persons. However, this is a premise for the convenience of description and whether the infected person and the contact person are in contact may be actually determined by a risk function.

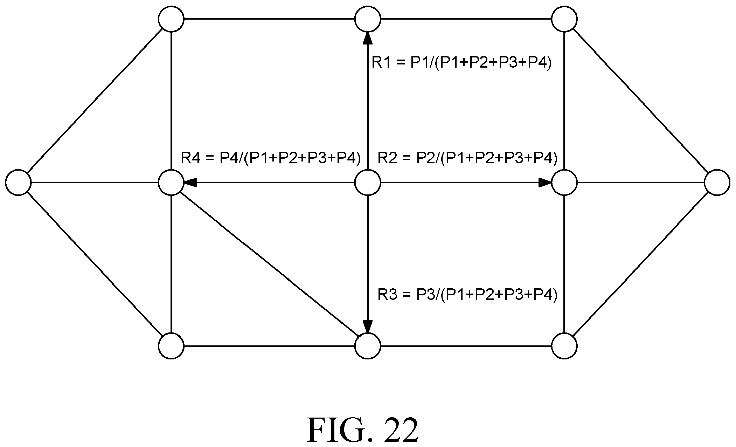

See FIG. 22. FIG. 22 illustrates a ratio of a disease spreading probability of a specific person located at the center, with respect to a plurality of contact persons who is in contact with a specific person, among the plurality of persons illustrated in FIG. 21.

For example, a ratio R1 of the disease spreading probability of the specific person with respect to the contact person located at the top is determined by a value {P1/(P1+P2+P3+P4)} obtained by dividing a disease spreading probability P1 between the specific person and the contact person located at the top by a sum P1+P2+P3+P4 of the disease spreading probabilities between the plurality of contact persons who is in contact with the specific person.

For example, a ratio R2 of the disease spreading probability of the specific person with respect to the contact person located at the right side is determined by a value {P2/(P1+P2+P3+P4)} obtained by dividing a disease spreading probability P2 between the specific person and the contact person located at the right side by a sum P1+P2+P3+P4 of the disease spreading probabilities between the plurality of contact persons who is in contact with the specific person.

For example, a ratio R3 of the disease spreading probability of the specific person with respect to the contact person located at the bottom is determined by a value {P3/(P1+P2+P3+P4)} obtained by dividing a disease spreading probability P3 between the specific person and the contact person located at the bottom by a sum P1+P2+P3+P4 of the disease spreading probabilities between the plurality of contact persons who is in contact with the specific person.

For example, a ratio R4 of the disease spreading probability of the specific person with respect to the contact person located at the left side is determined by a value {P4/(P1+P2+P3+P4)} obtained by dividing a disease spreading probability P4 between the specific person and the contact person located at the left side by a sum P1+P2+P3+P4 of the disease spreading probabilities between the plurality of contact persons who is in contact with the specific person.

In the meantime, a sum of the ratios of the disease spreading probability of some person, to a plurality of contact persons who is in contact with some person, with respect to some person is 1. For example, the sum of the ratios of the disease spreading probability of a specific person located at the center, to a plurality of contact persons who is in contact with the specific person illustrated in FIG. 7 is 1 {P1/(P1+P2+P3+P4) + P2/(P1+P2+P3+P4) + P3/(P1+P2+P3+P4) + P4/(P1+P2+P3+P4)}. As it will be described below, this considers that the disease may be spread to some people from any one of a plurality of contact persons who is in contact with some people.

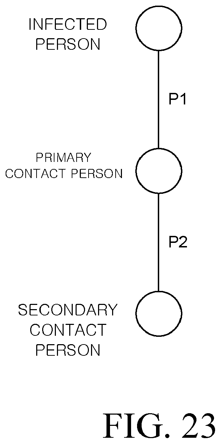

See FIG. 23. In FIG. 23, the primary contact person who is in contact with the infected person and the secondary contact person who is in contact with the primary contact person are illustrated. This is a premise for the convenience of description and whether the infected person is in contact with the primary contact person and whether the primary contact person is in contact with the secondary contact person may be actually determined by the risk function.

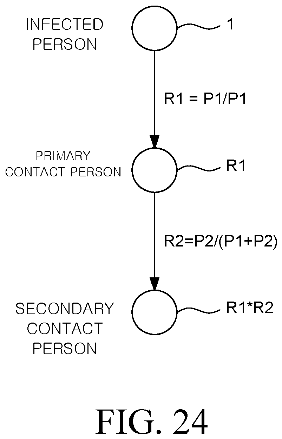

See FIG. 24. FIG. 24 is an exemplary diagram for explaining a method for calculating a probability of having a disease of a primary contact person and a probability of having a disease of a secondary contact person illustrated in FIG. 23.

The infected person is determined by the user so that under the premise that the infected person is an infected confirmed case, the probability of having a disease of the infected person is considered as 1.

For example, a ratio R1 of the disease spreading probability of the infected person to the primary contact person may be determined by a value (P1/P1 = 1) obtained by dividing the disease spreading probability P1 between the infected person and the primary contact person by a disease spreading probability P1 between the infected person and the primary contact person. At this time, the computing device 12 may determine a product (1^{∗}R1 = R1) of the probability (= 1) of having a disease of the infected person and a ratio R1 of the disease spreading probability of the infected person to the primary contact person as a probability of having a disease of the primary contact person.

For example, a ratio R2 of the disease spreading probability of the primary contact person to the secondary contact person may be determined by a value (P2/(P1+P2)) obtained by dividing the disease spreading probability P2 between the primary contact person and the secondary contact person by a sum P1+P2 of a disease spreading probability P1 between the primary contact person and the infected person and a disease spreading probability P2 between the primary contact person and the secondary contact person. At this time, the computing device 12 may determine a product (R1^{∗}R2) of the probability R1 of having a disease of the primary contact person and the ratio R2 of the disease spreading probability of the primary contact person to the secondary contact person as a probability of having a disease of the secondary contact person.

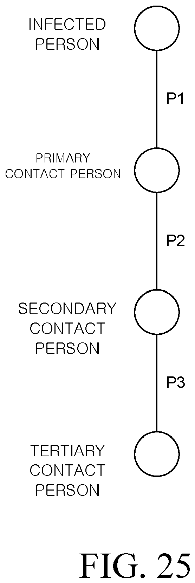

See FIG. 25. In FIG. 25, a primary contact person who is in contact with the infected person, a secondary contact person who is in contact with the primary contact person, and a tertiary contact person who is in contact with the secondary contact person are illustrated. This is a premise for the convenience of description and whether the infected person is in contact with the primary contact person, whether the primary contact person is in contact with the secondary contact person, and whether the secondary contact person is in contact with the tertiary contact person may be actually determined by the risk function.

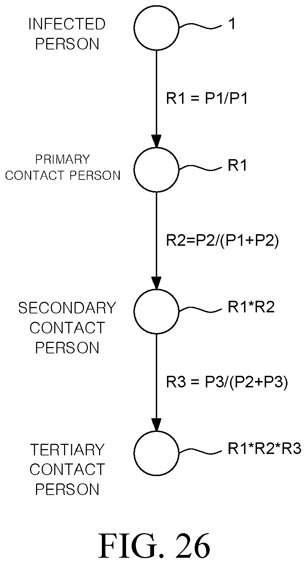

See FIG. 26. FIG. 26 is an exemplary diagram for explaining a method for calculating a probability of having a disease of a primary contact person, a probability of having a disease of a secondary contact person, and a probability of having a disease of a tertiary contact person illustrated in FIG. 25.

The infected person is determined by the user so that under the premise that the infected person is an infected confirmed case, the probability of having a disease of the infected person is considered as 1.

For example, a ratio R1 of the disease spreading probability of the infected person to the primary contact person may be determined by a value (P1/P1 = 1) obtained by dividing the disease spreading probability P1 between the infected person and the primary contact person by a disease spreading probability P1 between the infected person and the primary contact person. At this time, the computing device 12 may determine a product (1^{∗}R1) of the probability (= 1) of having a disease of the infected person and a ratio R1 of the disease spreading probability of the infected person to the primary contact person as a probability of having a disease of the primary contact person.

For example, a ratio R2 of the disease spreading probability of the primary contact person to the secondary contact person may be determined by a value (P2/(P1+P2)) obtained by dividing the disease spreading probability P2 between the primary contact person and the secondary contact person by a sum P1+P2 of a disease spreading probability P1 between the primary contact person and the infected person and a disease spreading probability P2 between the primary contact person and the secondary contact person. At this time, the computing device 12 may determine a product (R1^{∗}R2) of the probability R1 of having a disease of the primary contact person and the ratio R2 of the disease spreading probability of the primary contact person to the secondary contact person as a probability of having a disease of the secondary contact person.

For example, a ratio R3 of the disease spreading probability of the secondary contact person to the tertiary contact person may be determined by a value (P3/(P2+P3)) obtained by dividing the disease spreading probability P3 between the secondary contact person and the tertiary contact person by a sum P2+P3 of a disease spreading probability P2 between the primary contact person and the secondary contact person and a disease spreading probability P3 between the secondary contact person and the tertiary contact person. At this time, the computing device 12 may determine a product (R1^{∗}R2^{∗}R3) of the probability R1^{∗}R2 of having a disease of the secondary contact person and the ratio R3 of the disease spreading probability of the secondary contact person to the tertiary contact person as a probability of having a disease of the secondary contact person.

In the meantime, even though FIGS. 23 to 26 have been described with an example that there is only the secondary contact person or the tertiary contact person, This principle may be expanded to the case that there are a quaternary contact person who is in contact with the tertiary contact person, a fifth-order contact person who is in contact with the quaternary contact person, ..., and a n-th order contact person who is in contact with a n-1-th order contact person.

When there is a plurality of contact routes between the infected person and a specific person, the computing device 12 calculates a probability of having a disease of the specific person according to each of the plurality of contact routes and determines the maximum value of the probability of having a disease of the specific person calculated according to each of the plurality of contact routes as the probability of having a disease of the specific person.

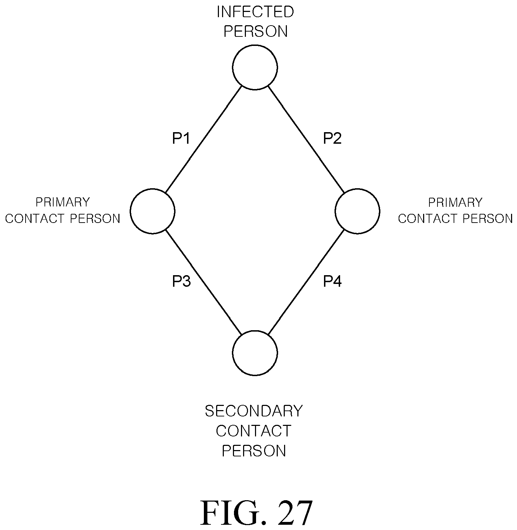

See FIG. 27. In FIG. 27, two primary contact persons who are in contact with the infected person and one secondary contact person (specific person) who is in contact with two primary contact persons are illustrated. This is a premise for the convenience of description and whether the infected person is in contact with two primary contact persons and whether two primary contact persons are in contact with the specific person may be actually determined by the risk function.

Referring to FIG. 27, there are two contact routes between the infected person and the specific person.

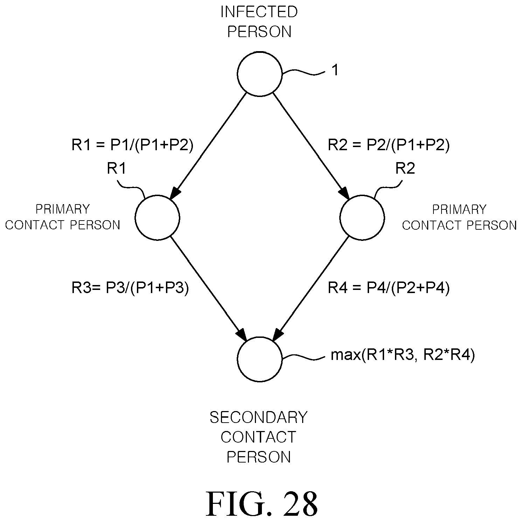

See FIG. 28. FIG. 28 is an exemplary diagram for explaining a method for calculating a probability of having a disease of two primary contact persons and a probability of having a disease of a specific person illustrated in FIG. 27.

The infected person is determined by the user so that under the premise that the infected person is an infected confirmed case, the probability of having a disease of the infected person is considered as 1.

For example, a ratio R1 of the disease spreading probability of the infected person to the primary contact person at the left side may be determined by a value {(P1/P1+P2)} obtained by dividing the disease spreading probability P1 between the infected person and the primary contact person at the left side by a sum P1+P2 of a disease spreading probability P1 between the infected person and the primary contact person at the left side and a disease spreading probability P2 between the infected person and the primary contact person at the right side. At this time, the computing device 12 may determine a product (1^{∗}R1 = R1) of the probability (= 1) of having a disease of the infected person and a ratio R1 of the disease spreading probability of the infected person to the primary contact person at the left side as a probability of having a disease of the primary contact person.

For example, a ratio R2 of the disease spreading probability of the infected person to the primary contact person at the right side may be determined by a value {(P2/P1+P2)} obtained by dividing the disease spreading probability P2 between the infected person and the primary contact person at the right side by a sum P1+P2 of a disease spreading probability P1 between the infected person and the primary contact person at the left side and a disease spreading probability P2 between the infected person and the primary contact person at the right side. At this time, the computing device 12 may determine a product (1^{∗}R2 = R2) of the probability (= 1) of having a disease of the infected person and a ratio R2 of the disease spreading probability of the infected person to the primary contact person at the right side as a probability of having a disease of the primary contact person.

For example, a ratio R3 of the disease spreading probability of the primary contact person at the left side to a specific person may be determined by a value {(P3/P1+P3)} obtained by dividing a disease spreading probability P3 between the primary contact person at the left side and the specific person by a sum P1+P3 of a disease spreading probability P1 between the primary contact person at the left side and the infected person and a disease spreading probability P3 between the primary contact person at the left side and the specific person. Further, a ratio R4 of the disease spreading probability of the primary contact person at the right side to a specific person may be determined by a value {(P4/P2+P4)} obtained by dividing a disease spreading probability P4 between the primary contact person at the right side and the specific person by a sum P2+P4. Here, P2 is a disease spreading probability between the primary contact person at the right side and the infected person and P4 is a disease spreading probability between the primary contact person at the right side and the specific person.

For example, the computing device 12 may determine a product (R1^{∗}R3) of the probability R1 of having a disease of the primary contact person at the left side and the ratio R3 of the disease spreading probability of the primary contact person at the left side to the specific person as a probability of having a disease of the specific person according to a left contact route. Further, the computing device 12 may determine a product (R2^{∗}R4) of the probability R2 of having a disease of the primary contact person at the right side and the ratio R4 of the disease spreading probability of the primary contact person at the right side to the specific person as a probability of having a disease of the specific person according to a right contact route. At this time, the computing device 12 may determine the maximum value {max (R1^{∗}R3, R2^{∗}R4)} between the probability R1^{∗}R3 of having a disease of the specific person according to the left contact route and the probability R2^{∗}R4 of having a disease of the specific person according to the right contact route as the probability of having a disease of the specific person.

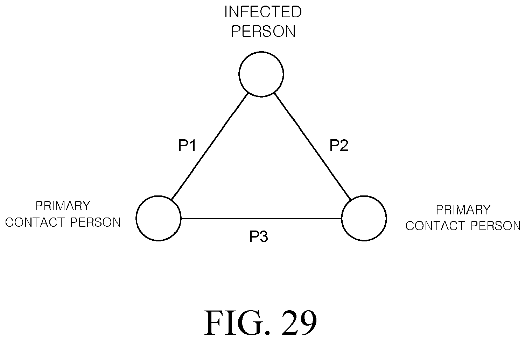

See FIG. 29. In FIG. 29, two primary contact persons who are in contact with the infected person are illustrated. Further, the primary contact person at the left side has been in contact with the primary contact person at the right side. This is a premise for the convenience of description and whether the infected person is in contact with two primary contact persons and whether two primary contact persons are in contact with each other may be actually determined by the risk function.

Referring to FIG. 29, there are two contact routes between the infected person and each of the primary contact persons. In the meantime, the primary contact person at the left side has been in direct contact with the infected person and is in indirect contact with the infected person via the primary contact person at the right side. Therefore, the primary contact person at the left side simultaneously becomes a primary contact person and a secondary contact person. Further, the primary contact person at the right side has been in direct contact with the infected person and has been in indirect contact with the infected person via the primary contact person at the left side. Therefore, the primary contact person at the right side simultaneously becomes a primary contact person and a secondary contact person.

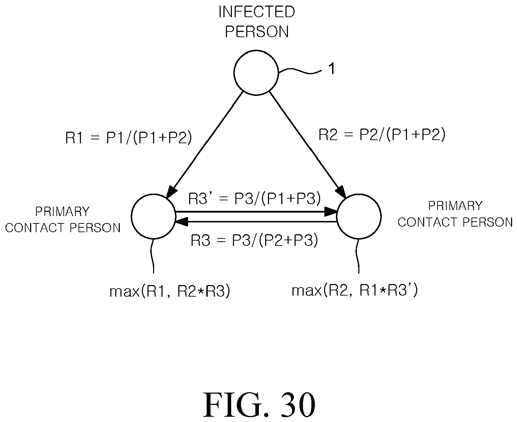

See FIG. 30. FIG. 30 is an exemplary diagram for explaining a method for calculating a probability of having a disease of two primary contact persons (specific persons) illustrated in FIG. 29.

The infected person is determined by the user so that under the premise that the infected person is an infected confirmed case, the probability of having a disease of the infected person is considered as 1.

For example, a ratio R1 of the disease spreading probability of the infected person to the primary contact person at the left side may be determined by a value {(P1/P1+P2)} obtained by dividing the disease spreading probability P1 between the infected person and the primary contact person at the left side by a sum P1+P2 of a disease spreading probability P1 between the infected person and the primary contact person at the left side and a disease spreading probability P2 between the infected person and the primary contact person at the right side. Further, a ratio R2 of the disease spreading probability of the infected person to the primary contact person at the right side may be determined by a value {(P2/P1+P2)} obtained by dividing the disease spreading probability P2 between the infected person and the primary contact person at the right side by a sum P1+P2 of a disease spreading probability P1 between the infected person and the primary contact person at the left side and a disease spreading probability P2 between the infected person and the primary contact person at the right side.

For example, the ratio R3 of the disease spreading probability of the primary contact person at the right side to the primary contact person at the left side may be determined by a value {P3/(P2+P3)} obtained by dividing the disease spreading probability P3 between the primary contact person at the left side and the primary contact person at the right side by a sum P2+P3 of the disease spreading probability P2 between the primary contact person at the right side and the infected person and the disease spreading probability P3 between the primary contact person at the right side and the primary contact person at the left side. Further, the ratio R3' of the disease spreading probability of the primary contact person at the left side to the primary contact person at the right side may be determined by a value {P3/(P1+P3)} obtained by dividing the disease spreading probability P3 between the primary contact person at the right side and the primary contact person at the left side by a sum P1+P3 of the disease spreading probability P1 between the primary contact person at the left side and the infected person and the disease spreading probability P3 between the primary contact person at the left side and the primary contact person at the right side.

At this time, the computing device 12 may determine a product (1^{∗}R1 = R1) of the probability (= 1) of having a disease of the infected person and a ratio R1 of the disease spreading probability of the infected person to the primary contact person at the left side as a probability of having a disease of the primary contact person at the left side according to the left contact route. Further, the computing device 12 may determine a product (1^{∗}R2^{∗}R3 = R2^{∗}R3) of the probability (= 1) of having a disease of the infected person, a ratio R2 of the disease spreading probability of the infected person to the primary contact person at the right side, and a ratio R3 of the disease spreading probability of the primary contact person at the right side to the primary contact person at the left side as a probability of having a disease of the primary contact person at the left side according to the right contact route. Further, the computing device 12 determines the maximum value {max (R1, R2^{∗}R3)} between the probability R1 of having a disease of the primary contact person at the left side according to the left contact route and the probability R2^{∗}R3 of having a disease of the primary contact person at the left side according to the right contact route as the probability of having a disease of the primary contact person at the left side.

In the meantime, the computing device 12 may determine a product (1^{∗}R2 = R2) of the probability (= 1) of having a disease of the infected person and a ratio R2 of the disease spreading probability of the infected person to the primary contact person at the right side as a probability of having a disease of the primary contact person at the right side according to the right contact route. Further, the computing device 12 may determine a product (1^{∗}R1^{∗}R3' = R1^{∗}R3') of the probability (= 1) of having a disease of the infected person, a ratio R1 of the disease spreading probability of the infected person to the primary contact person at the left side, and a ratio R3' of the disease spreading probability of the primary contact person at the left side to the primary contact person at the right side as a probability of having a disease of the primary contact person at the right side according to the left contact route. Further, the computing device 12 determines the maximum value {max (R2, R1^{∗}R3')} between the probability R2 of having a disease of the primary contact person at the right side according to the right contact route and the probability R1^{∗}R3' of having a disease of the primary contact person at the right side according to the left contact route as the probability of having a disease of the primary contact person at the right side.

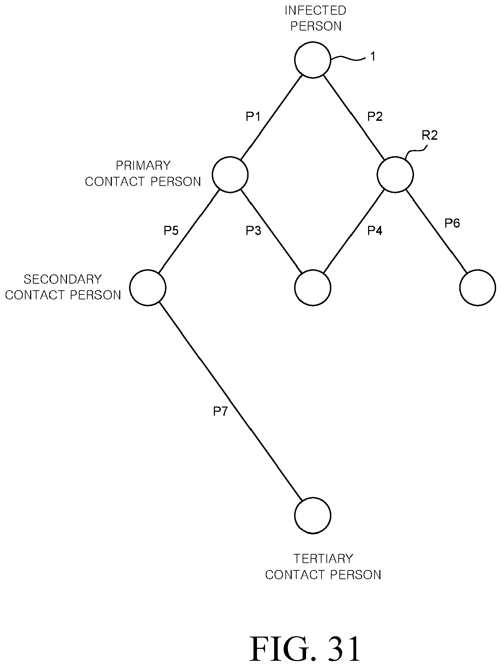

See FIG. 31. In FIG. 31, one infected person, two primary contact persons who are in contact with the infected person, three secondary contact persons who are in contact with the primary contact person, and one tertiary contact person who is in contact with the secondary contact person are illustrated. Further, the secondary contact person at the center among three secondary contact persons has been in contact with the primary contact person at the left side and the primary contact person at the right side. This is a premise for the convenience of description and whether the infected person is in contact with two primary contact persons, whether two primary contact persons are in contact with three secondary contact persons, whether a secondary contact person at the center and the primary contact person at the left side are in contact with each other, whether a secondary contact person at the center and the primary contact person at the right side are in contact with each other, and whether the secondary contact person at the left side and the tertiary contact person are in contact with each other may be actually determined by the risk function.

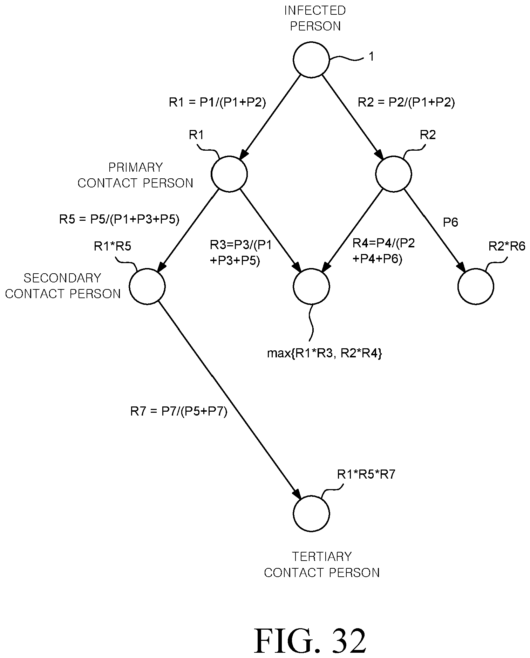

See FIG. 32. FIG. 32 is an exemplary diagram for explaining a method for calculating a probability of having a disease of two primary contact persons, a probability of having a disease of three secondary contact persons, and a probability of having a disease of a tertiary contact person illustrated in FIG. 31.

The infected person is determined by the user so that under the premise that the infected person is an infected confirmed case, the probability of having a disease of the infected person is considered as 1.

For example, a ratio R1 of the disease spreading probability of the infected person to the primary contact person at the left side may be determined by a value {(P1/P1+P2)} obtained by dividing the disease spreading probability P1 between the infected person and the primary contact person at the left side by a sum P1+P2 of a disease spreading probability P1 between the infected person and the primary contact person at the left side and a disease spreading probability P2 between the infected person and the primary contact person at the right side. At this time, the computing device 12 may determine a product (1^{∗}R1 = R1) of the probability (= 1) of having a disease of the infected person and a ratio R1 of the disease spreading probability of the infected person to the primary contact person at the left side as a probability of having a disease of the primary contact person.

For example, a ratio R2 of the disease spreading probability of the infected person to the primary contact person at the right side may be determined by a value {(P2/P1+P2)} obtained by dividing the disease spreading probability P2 between the infected person and the primary contact person at the right side by a sum P1+P2 of a disease spreading probability P1 between the infected person and the primary contact person at the left side and a disease spreading probability P2 between the infected person and the primary contact person at the right side. At this time, the computing device 12 may determine a product (1^{∗}R2 = R2) of the probability (= 1) of having a disease of the infected person and a ratio R2 of the disease spreading probability of the infected person to the primary contact person at the right side as a probability of having a disease of the primary contact person.

For example, the ratio R3 of the disease spreading probability of the primary contact person at the left side to the secondary contact person at the center may be determined by a value {P3/(P1+P3+P5)} obtained by dividing the disease spreading probability P3 between the primary contact person at the left side and the secondary contact person at the center by a sum P1+P3+P5. Here, P1 is the disease spreading probability between the primary contact person at the left side and the infected person, P3 is the disease spreading probability between the primary contact person at the left side and the secondary contact person at the center, and P5 is a disease spreading probability between the primary contact person at the left side and the secondary contact person at the left side. Further, a ratio R4 of the disease spreading probability of the primary contact person at the right side to the secondary contact person at the center may be determined by a value {P4/(P2+P4+P6)} obtained by dividing the disease spreading probability P4 between the primary contact person at the right side and the secondary contact person at the center by a sum P2+P4+P6. Here, P2 is a disease spreading probability P2 between the primary contact person at the right side and the infected person, P4 is a disease spreading probability between the primary contact person at the right side and the secondary contact person at the center, and P6 is a disease spreading probability between the primary contact person at the right side and the secondary contact person at the right side.

At this time, the computing device 12 may determine a product (R1^{∗}R3) of the probability R1 of having a disease of the primary contact person at the left side and the ratio R3 of the disease spreading probability of the primary contact person at the left side to the secondary contact person at the center as a probability of having a disease of the secondary contact person at the center according to the left contact route. Further, the computing device 12 may determine a product (R2^{∗}R4) of the probability R2 of having a disease of the primary contact person at the right side and the ratio R4 of the disease spreading probability of the primary contact person at the right side to the secondary contact person at the center as a probability of having a disease of the secondary contact person at the center according to the right contact route. Further, the computing device 12 may determine the maximum value {max (R1^{∗}R3, R2^{∗}R4)} between the probability R1^{∗}R3 of having a disease of the secondary contact person at the center according to the left contact route and the probability R2^{∗}R4 of having a disease of the secondary contact person at the center according to the right contact route as the probability of having a disease of the secondary contact person at the center.

For example, a ratio R5 of the disease spreading probability of the primary contact person at the left side to the secondary contact person at the left side may be determined by a value {P5/(P1+P3+P5)} obtained by dividing the disease spreading probability P5 between the primary contact person at the left side and the secondary contact person at the left side by a sum P1+P3+P5. Here, P1 is the disease spreading probability between the primary contact person at the left side and the infected person, P3 is the disease spreading probability between the primary contact person at the left side and the secondary contact person at the center, P5 is a disease spreading probability between the primary contact person at the left side and the secondary contact person at the left side. At this time, the computing device 12 may determine a product (1^{∗}R1^{∗}R5 = R1^{∗}R5) of the probability (= 1) of having a disease of the infected person, a ratio R1 of the disease spreading probability of the infected person to the primary contact person at the left side, and a ratio R5 of the disease spreading probability of the primary contact person at the left side to the secondary contact person at the left side as a probability of having a disease of the secondary contact person at the left side.

For example, the ratio R6 of the disease spreading probability of the primary contact person at the right side to the secondary contact person at the right side may be determined by a value {P6/(P2+P4+P6)} obtained by dividing the disease spreading probability P6 between the primary contact person at the right side and the secondary contact person at the right side by a sum P2+P4+P6. Here, P2 is the disease spreading probability between the primary contact person at the right side and the infected person, P4 is the disease spreading probability between the primary contact person at the right side and the secondary contact person at the center, and P6 is a disease spreading probability between the primary contact person at the right side and the secondary contact person at the right side. At this time, the computing device 12 may determine a product (1^{∗}R2^{∗}R6 = R2^{∗}R6) of the probability (= 1) of having a disease of the infected person, a ratio R2 of the disease spreading probability of the infected person to the primary contact person at the right side, and a ratio R6 of the disease spreading probability of the primary contact person at the right side to the secondary contact person at the right side as a probability of having a disease of the secondary contact person at the right side.

For example, a ratio R7 of the disease spreading probability of the secondary contact person to the tertiary contact person may be determined by a value {P7/(P5+P7)} obtained by dividing the disease spreading probability P7 between the secondary contact person at the left side and the tertiary contact person by a sum P5+P7. Here, P5 is a disease spreading probability between the secondary contact person at the left side and the primary contact person at the left side and P7 is a disease spreading probability between the secondary contact person at the left side and the tertiary contact person. At this time, the computing device 12 may determine a product (1^{∗}R1^{∗}R5^{∗}R7 = R1^{∗}R5^{∗}R7) of the probability (= 1) of having a disease of the infected person, a ratio R1 of the disease spreading probability of the infected person to the primary contact person at the left side, a ratio R5 of the disease spreading probability of the primary contact person at the left side to the secondary contact person at the left side, and a ratio R7 of a disease spreading probability between the secondary contact person at the left side to the tertiary contact person as a probability of having a disease of the tertiary contact person.

When there is a plurality of infected persons, the computing device 12 may independently calculate a probability of having a disease of each of the plurality of persons in consideration of a disease spreading effect by each of the plurality of infected persons. The computing device 12 may independently determines an arithmetic mean value of the probabilities of having a disease of the plurality of persons. Here, the arithmetic mean value 12 is independently calculated in consideration of the disease spreading effect by each of the plurality of infected persons. Thus, the computing device 12 may calculate a probability of having a disease of each of the plurality of persons.

For example, since two infected persons are a first infected person and a second infected person, the computing device 12 may calculate a probability of being infected with a disease of each of the plurality of persons in consideration of only the disease spreading effect by the first infected person. The computing device may calculate a probability of having a disease of each of the plurality of persons in consideration of only the disease spreading effect by the second infected person. And then, the computing device 12 may determine the probability of having a disease of each of the plurality of persons as an arithmetic mean value of the two previous probabilities of having a disease of each of the plurality of persons.

In the meantime, when the probability of having a disease of each of the plurality of persons is calculated in consideration of only the disease spreading effect by the first infected person. It means that when the probability of having a disease of each of the plurality of persons is calculated in consideration of only the disease spreading effect by the first infected person, there is no disease spreading effect of an infected person (the second infected person) other than the first infected person. Further, when the probability of having a disease of each of the plurality of persons is calculated in consideration of only the disease spreading effect by the second infected person. It means that when the probability of having a disease of each of the plurality of persons is calculated in consideration of only the disease spreading effect by the second infected person, there is no disease spreading effect of an infected person (the first infected person) other than the second infected person. By expanding this, it is possible to independently calculate the probability of having a disease of each of the plurality of persons in consideration of the disease spreading effect by each of the plurality of infected persons.

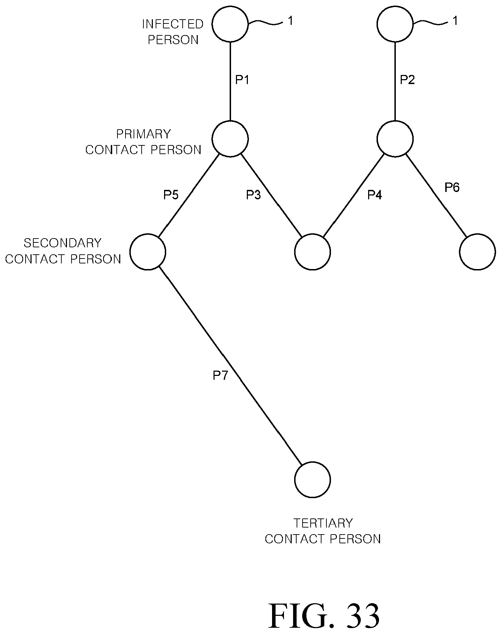

See FIG. 33. In FIG. 33, two infected persons, two primary contact persons who are in contact with two infected persons, four secondary contact persons who are in contact with the primary contact persons, and one tertiary contact person who is in contact with the secondary contact person are illustrated. This is a premise for the convenience of description and whether two infected persons are in contact with two primary contact persons, whether two primary contact persons are in contact with four secondary contact persons, and whether the secondary contact person at the left side is in contact with the tertiary contact person may be actually determined by the risk function.

Since two infected persons are a first infected person and a second infected person, the computing device 12 may calculates a probability of having a disease of each of the plurality of persons in consideration of only the disease spreading effect by the first infected person. The computing device 12 may calculate a probability of having a disease of each of the plurality of persons in consideration of only the disease spreading effect by the second infected person. The computing device 12 may determine an arithmetic mean value of the probability of having a disease of each of the plurality of persons calculated and the probability of having a disease of each of the plurality of persons calculated as a probability of having a disease of each of the plurality of persons.

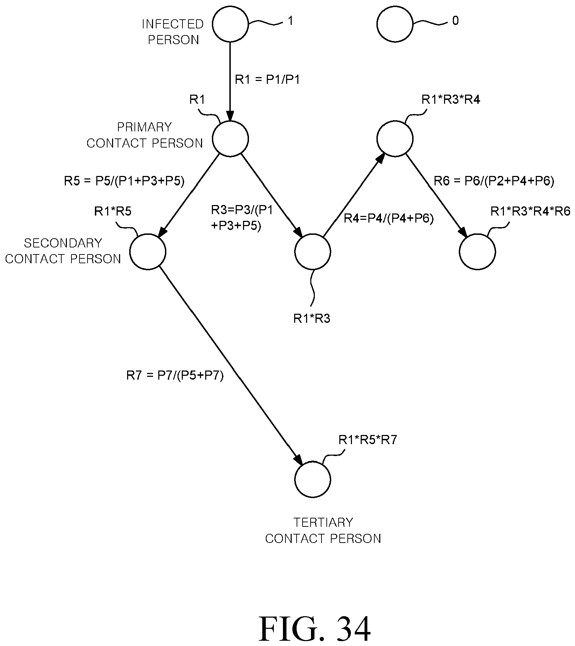

FIG. 34 illustrates a probability of having a disease of each of the plurality of persons calculated in consideration of only the disease spreading effect by the first infected person in FIG. 33. At this time, the probability of having a disease of the second infected person is considered to be zero so that it may be ignored. Further, FIG. 20 illustrates a probability of having a disease of each of the plurality of persons calculated in consideration of only the disease spreading effect by the second infected person in FIG. 18. At this time, the probability of having a disease of the first infected person is considered to be zero so that it may be ignored. FIG. 21 illustrates an arithmetic mean value of the probability of having a disease of each of the plurality of persons calculated in consideration of only the disease spreading effect by the first infected person (see FIG. 19) and the probability of having a disease of each of the plurality of persons calculated in consideration of only the disease spreading effect by the second infected person (see FIG. 20).

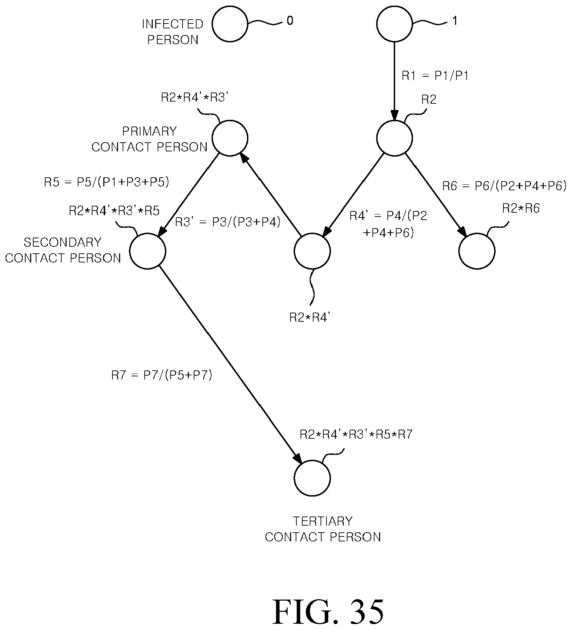

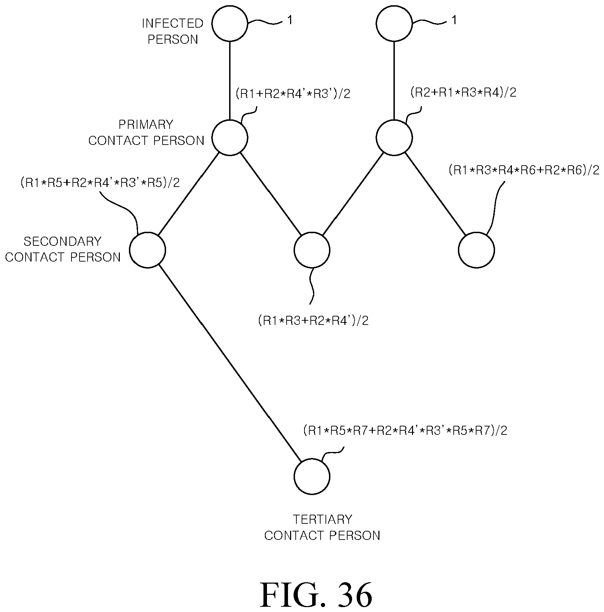

By expanding this, unlike FIGS. 33 to 36, when there are a total of n infected persons including the first infected person, the second infected person to an n-th infected person, the computing device 12 may independently calculate the probability of having a disease of each of the plurality of persons in consideration of the disease spreading effect by each of the plurality of infected persons. The computing device 12 may determine the arithmetic mean value of the probability of having a disease of each of the plurality of persons as a probability of having a disease of each of the plurality of persons. Here, the arithmetic mean value is independently calculated in consideration of the disease spreading effect by each of the plurality of infected persons. Thus, the computing device 12 may calculate a probability of having a disease of each of the plurality of persons.

By doing this, the computing device 12 may calculate the probability of having a disease of each of the plurality of persons only with the infected person information input by the user.

The computing device 12 determines a person who is estimated to have a disease among the plurality of persons on the basis of the probability of having a disease of each of the plurality of persons.

According to an exemplary embodiment, the computing device 12 receives a searching period from the user and determines a person who is estimated to have a disease among the plurality of persons on the basis of the location information of each of the plurality of persons corresponding to the searching period. For example, the computing device 12 receives the searching period from the user, calculates a probability of having a disease of each of the plurality of persons on the basis of the information of the infected person and the location information of each of the plurality of persons corresponding to the searching period, and determines a person who is estimated to have a disease among the plurality of persons on the basis of the probability of having a disease of each of the plurality of persons. By doing this, when the person who is estimated to have a disease is determined, an inflow timing of the infected person or a disease onset timing is considered to reduce an unnecessary computation amount. For example, the user inputs the inflow timing of the infected person or the disease onset timing as a starting timing of the searching period and the current timing as an end timing of the searching period.

According to the exemplary embodiment, the computing device 12 determines a disease latent period corresponding to disease information of the infected person and sets the searching period on the basis of the disease latent period. For example, the computing device may 12 store a latent period table for every disease and determines a disease latent period corresponding to the disease information of the infected person on the basis of the latent period table for every disease. Thereafter, the computing device 12 may determine the person who is estimated to have a disease among the plurality of persons on the basis of the location information of each of the plurality of persons corresponding to the searching period. For example, the computing device 12 may receive the searching period from the user, calculates a probability of having a disease of each of the plurality of persons on the basis of the information of the infected person and the location information of each of the plurality of persons corresponding to the searching period. The computing device 12 may determine a person who is estimated to have a disease among the plurality of persons on the basis of the probability of having a disease of each of the plurality of persons.

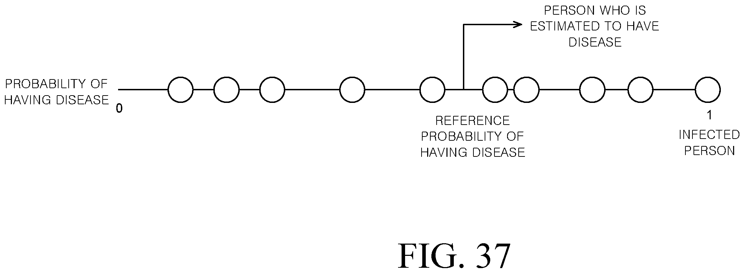

As illustrated in FIG. 37, the computing device 12 may determine a person whose probability of having a disease exceeds a predetermined reference probability of having a disease, among the plurality of persons, as a person who is estimated to have a disease. In the meantime, the user inputs the reference probability of having a disease to the computing device 12 to set the reference probability of having a disease.

According to an exemplary embodiment, the computing device 12 may output information related to the person who is estimated to have a disease. For example, the computing device 12 may visually or acoustically output information related to the person who is estimated to have a disease. For example, the computing device 12 may output identification information of the person who is estimated to have a disease.

FIG. 38 is a block diagram for explaining a computing environment including a computing device suitable to be used in exemplary embodiments.

In the illustrated exemplary embodiment, each component has different function and ability other than those described below and includes additional component as well as those described below.

The illustrated computing environment 10 includes a computing device 12. The computing device 12 includes at least one processor 14, a computer readable storage medium 16, and a communication bus 18. The processor 14 may cause the computing device 12 to operate according to the above-mentioned exemplary embodiment. For example, the processor 14 may execute one or more programs stored in the computer readable storage medium 16. One or more programs may include one or more computer executable instructions and the computer executable instruction may be configured to allow the computing device 12 to perform the operations according to the exemplary embodiments when it is executed by the processor 14.

The computer readable storage medium 16 is configured to store a computer executable instruction or program code, program data and/or other appropriate formats of information. The program 20 stored in the computer readable storage medium 16 includes a set of instructions executable by the processor 14. In one exemplary embodiment, the computer readable storage medium 16 may be a memory (a volatile memory such as a random-access memory, a non-volatile memory, or an appropriate combination thereof), one or more magnetic disk storage devices, optical disk storage devices, flash memory devices, and another format of storage mediums accessed by the computing device 12 and stores desired information, or an appropriate combination thereof.

The communication bus 18 includes a processor 14 and a computer readable storage medium 16 to interconnect various components of the computing device 12 to each other.

The computing device 12 may include one or more input/output interfaces 22 and one or more network communication interfaces 26 providing an interface for one or more input/output devices 24. The input/output interface 22 and the network communication interface 26 are connected to the communication bus 18. The input/output device 24 may be connected to the other components of the computing device 12 by means of the input/output interface 22. The exemplary input/output device 24 may include an input device such as a pointing device (a mouse, a track pad, or the like), a keyboard, a touch input device (a touch pad, a touch screen, or the like), a voice or sound input device, various types of sensor devices and/or an image capturing device, and/or an output device such as a display device, a printer, a speaker, and/or a network card. The exemplary input/output device 24 may be included in the computing device 12 as one component constituting the computing device 12 and connected to the computing device 12 as a separate device distinguished from the computing device 12.

Although the exemplary embodiments of the present invention have been described in detail with reference to the accompanying drawings, the present invention is not limited thereto and may be embodied in many different forms without departing from the technical concept of the present invention. Therefore, the exemplary embodiments of the present invention are provided for illustrative purposes only but not intended to limit the technical concept of the present invention. The scope of the technical concept of the present invention is not limited thereto. Therefore, it should be understood that the above-described exemplary embodiments are illustrative in all aspects and do not limit the present invention. The protective scope of the present invention should be construed based on the following claims, and all the technical concepts in the equivalent scope thereof should be construed as falling within the scope of the present invention.
[National R&D Project which supports this invention]
[Project Identification Number] 2018R1D1A1B07048179
[Government Department] Ministry of Education
[Project management (specialty) organization name] National Research Foundation of Korea
[Research Project Name] Individual Basic Research support project in Science and Engineering
[Research Title] Development of system for generating residual tumor risk map and predicting prognosis using deep learning analysis of multi-sequence fusion images in MRI image after chemotherapy of rectal cancer
[Contribution Rate] 1/2
[Supervising Organization] Yonsei University
[Research Period] 2019.03.01 to 2020.02.29
[National R&D Project which supports this invention]
[Project Identification Number] 2019R1A2C4069598
[Government Department] Ministry of Science and ICT
[Project management (specialty) organization name] National Research Foundation of Korea
[Research Project Name] Mid-level researcher support project
[Research Title] Investigation of integrative working memory specific EEG marker for mental disorders: Neuromodulation based study
[Contribution Rate] 1/2
[Supervising Organization] Yonsei University
[Research Period] 2019.06.01 to 2020.02.29

## Claims

1. A method carried out on a computing device with one or more processors and a memory storing one or more programs executed by the one or more processors, the method comprising the steps of:
receiving location information of each of a plurality of persons; and
calculating a probability of being infected with a disease for each of the plurality of persons, on the basis of infected person information including identification information thereof and disease information thereof, and location information of each of the plurality of persons.

2. The method of claim 1, wherein the step of calculating a probability of being infected with a disease for each of the plurality of persons includes the steps of:
calculating a disease spreading probability between the plurality of persons, on the basis of the infected person information and the location information of each of the plurality of persons; and
calculating a probability of being infected with a disease for each of the plurality of persons, on the basis of the disease spreading probability between the plurality of persons.

3. The method of claim 2, wherein the step of calculating a disease spreading probability between the plurality of persons includes the steps of:
determining a disease spreading power and a risk function corresponding to disease information of the infected person; and
calculating a disease spreading probability between the plurality of persons, on the basis of the disease spreading power, the risk function, and a contact degree between the plurality of persons.

4. The method of claim 3, wherein the step of calculating a disease spreading probability between the plurality of persons includes the steps of:
calculating a contact degree between the plurality of persons, on the basis of the risk function and the location information of each of the plurality of persons; and
calculating a disease spreading probability between the plurality of persons, on the basis of the disease spreading power and the contact degree between the plurality of persons.

5. The method of claim 1, wherein when there is a primary contact person who is in contact with the infected person and a secondary contact person who is in contact with the primary contact person,
the step of calculating a probability of being infected with a disease for each of the plurality of persons includes the steps of:
calculating a probability of being infected with a disease of the primary contact person; and
calculating a probability of being infected with a disease of the secondary contact person, on the basis of the probability of being infected with a disease of the primary contact person and a disease spreading probability between the primary contact person and the secondary contact person.

6. The method of claim 1, wherein the step of calculating a probability of being infected with a disease for each of the plurality of persons includes the steps of:
when there is a plurality of contact routes between the infected person and a specific person,
calculating a probability of being infected with a disease of the specific person according to each of the plurality of contact routes; and
determining a maximum value of the probability of being infected with a disease of the specific person calculated according to each of the plurality of contact routes as a probability of being infected with a disease of the specific person.

7. The method of claim 1, wherein the step of calculating a probability of being infected with a disease for each of the plurality of persons includes the steps of:
when there is the plurality of infected persons,
independently calculating the probability of being infected with a disease of each of the plurality of persons, in consideration of a disease spreading effect by each of the plurality of infected persons; and
determining an arithmetic mean value of the probability of being infected with a disease of each of the plurality of persons, as a probability of being infected with a disease of each of the plurality of persons;
wherein the arithmetic mean value is independently calculated in consideration of the disease spreading effect by each of the plurality of infected persons.

8. A device, comprising:
one or more processors;
a memory; and
one or more programs,
wherein the one or more programs are configured to be stored in the memory and executed by the one or more processors, and
the program includes instructions to execute the steps of:
receiving location information of each of a plurality of persons; and
calculating a probability of being infected with a disease for each of the plurality of persons, on the basis of infected person information including identification information thereof and disease information thereof, and location information of each of the plurality of persons.

9. A method carried out on a computing device with one or more processors and a memory storeing one or more programs executed by the one or more processors, the method comprising the steps of:
receiving location information of each of a plurality of persons;
receiving infected person information including identification information of an infected person and disease information of the infected person from a user;
determining a subject to be tested for a disease among the plurality of persons, on the basis of the disease information of the infected person and the location information of each of the plurality of persons; and
outputting information related to the subject to be tested for a disease.

10. The method of claim 9, further comprising a step of:
receiving a searching period from the user,
wherein in the step of determining a subject to be tested for a disease, the subject to be tested for a disease among the plurality of persons is determined on the basis of the location information of each of the plurality of persons corresponding to the searching period.

11. The method of claim 9, further comprising the steps of:
determining a disease latent period corresponding to the disease information of the infected person; and
setting a searching period on the basis of the disease latent period,
wherein in the step of determining a subject to be tested for a disease, the subject to be tested for a disease among the plurality of persons is determined on the basis of the location information of each of the plurality of persons corresponding to the searching period.

12. A device, comprising:
one or more processors;
a memory; and
one or more programs,
wherein the one or more programs are configured to be stored in the memory and executed by the one or more processors, and
the program includes instructions to execute the steps of:
receiving location information of each of a plurality of persons;
receiving infected person information including identification information of an infected person and disease information of the infected person from a user;
determining a subject to be tested for a disease among the plurality of persons, on the basis of the disease information of the infected person and the location information of each of the plurality of persons; and
outputting information related to the subject to be tested for a disease.

13. A method carried out on a computing device with one or more processors and a memory storing one or more programs executed by the one or more processors, the method comprising the steps of:
receiving location information of each of a plurality of persons;
calculating a disease spreading probability between the plurality of persons, on the basis of infected person information including identification information thereof and disease information thereof, and location information of each of the plurality of persons;
calculating a ratio of a disease spreading probability between the plurality of persons and contact persons who are in contact with the plurality of persons, with respect to each of the plurality of persons; and
calculating a probability of having a disease of each of the plurality of persons, on the basis of the ratio of the disease spreading probability between the plurality of persons and contact persons who are in contact with the plurality of persons.

14. The method of claim 13, wherein when there is a plurality of contact persons who is in contact with a specific person among the plurality of persons, a ratio of a disease spreading probability of the specific person to the specific contact person among the plurality of contact persons is calculated on the basis of a value obtained by dividing a disease spreading probability between the specific person and the specific contact person by a sum of a disease spreading probability between the specific person and the plurality of contact persons.

15. The method of claim 13, wherein the step of calculating a disease spreading probability between the plurality of persons includes the steps of:
determining a disease spreading power and a risk function corresponding to disease information of the infected person; and
calculating a disease spreading probability between the plurality of persons, on the basis of the disease spreading power, the risk function, and a contact degree between the plurality of persons.

16. The method of claim 15, wherein the step of calculating a disease spreading probability between the plurality of persons includes the steps of:
calculating a contact degree between the plurality of persons on the basis of the risk function and the location information of each of the plurality of persons; and
calculating a disease spreading probability between the plurality of persons, on the basis of the disease spreading power and the contact degree between the plurality of persons.

17. The method of claim 13, wherein when there is a primary contact person who is in contact with the infected person and a secondary contact person who is in contact with the primary contact person, the step of calculating a probability of having a disease for each of the plurality of persons includes the steps of:
calculating a probability of having a disease of the primary contact person; and
calculating a probability of having a disease of the secondary contact person, on the basis of the probability of having a disease of the primary contact person and a ratio of the disease spreading probability of the primary contact person to the secondary contact person.

18. The method of claim 13, wherein the step of calculating a probability of having a disease for each of the plurality of persons includes the steps of:
when there is a plurality of contact routes between the infected person and a specific person,
calculating a probability of having a disease of the specific person according to each of the plurality of contact routes; and
determining a maximum value of the probability of having a disease of the specific person calculated according to each of the plurality of contact routes as a probability of having a disease of the specific person.

19. The method of claim 13, wherein the step of calculating a probability of having a disease for each of the plurality of persons includes the steps of:
when there is the plurality of infected persons,
independently calculating the probability of having a disease of each of the plurality of persons in consideration of a disease spreading effect by each of the plurality of infected persons; and
determining an arithmetic mean value of the probability of having a disease of each of the plurality of persons, as a probability of having a disease of each of the plurality of persons;
wherein the arithmetic mean value is independently calculated in consideration of the disease spreading effect by each of the plurality of infected persons.

20. The method of claim 13, further comprising the steps of:
determining a person who is estimated to have a disease among the plurality of persons, on the basis of the probability of having a disease of each of the plurality of persons; and
outputting information related to the person who is estimated to have a disease.

21. The method of claim 20, further comprising a step of:
receiving a searching period from a user,
wherein in the step of calculating a disease spreading probability between the plurality of persons, the person who is estimated to have a disease among the plurality of persons is determined on the basis of the location information of each of the plurality of persons corresponding to the searching period.

22. The method of claim 20, further comprising: the steps of:
determining a disease latent period corresponding to the disease information of the infected person; and
setting a searching period on the basis of the disease latent period,
wherein in the step of calculating a disease spreading probability between the plurality of persons, the person who is estimated to have a disease among the plurality of persons is determined on the basis of the location information of each of the plurality of persons corresponding to the searching period.

23. A device, comprising:
one or more processors;
a memory; and
one or more programs,
wherein the one or more programs are configured to be stored in the memory and executed by the one or more processors, and
the program includes instructions to execute the steps of:
receiving location information of each of a plurality of persons;
calculating a disease spreading probability between the plurality of persons, on the basis of infected person information including identification information thereof and disease information thereof, and location information of each of the plurality of persons;
calculating a ratio of a disease spreading probability between the plurality of persons and contact persons who are in contact with the plurality of persons, with respect to each of the plurality of persons; and
calculating a probability of having a disease of each of the plurality of persons, on the basis of a ratio of the disease spreading probability between the plurality of persons and contact persons who are in contact with the plurality of persons.
